# EUROPEAN PATENT SPECIFICATION

(11) **EP 0 818 123 B1**
(45) Date of publication and mention of the grant of the patent: **15.01.2003**
(21) Application number: 96907216.4
(22) Date of filing: 29.03.1996
(51) Int. Cl.: H04R 25/00, A61F 2/18, A61F 11/04, A61M 25/01, A61M 25/088, A61M 25/09, A61N 1/05

(54) **COCHLEAR ELECTRODE IMPLANT ASSEMBLIES WITH POSITIONING SYSTEM THEREFOR**
ELEKTRODENANORDNUNGEN FÜR COCHLEARES IMPLANTAT UND POSITIONIERUNGSSYSTEM
ENSEMBLE ELECTRODES COCHLEAIRES IMPLANTES ET LEUR SYSTEME DE POSITIONNEMENT

(30) Priority: 30.03.1995 US 414656; 15.03.1996 US 616299
(43) Date of publication of application: 14.01.1998
(73) Proprietor: Cochlear Limited, Lane Cove, NSW 2066 (AU)
(72) Inventor: KUZMA, Janusz, Englewood, CO 80111 (US)
(74) Representative: Molyneaux, Martyn William
(86) International application number: AU9600179
(87) International publication number: WO96031087

(56) References cited:
- EP-A- 0 002 068
- WO-A-93/06698
- DE-A- 4 200 030
- US-A- 5 370 679

## Description

### Technical Field

This invention relates to cochlear electrode implant assemblies, and more particularly to a system for optimally positioning an implanted stimulating electrode assembly including an array of electrodes on a carrier therefor in the cochlea of a human ear.

### Background of the Invention

The present invention is an improvement over the cochlear electrode implant and in particular over the cochlear electrode positioning system disclosed in PCT/AU92/00517 (published as WO93/06698) according to which the preamble of claim 1 has been delimited.

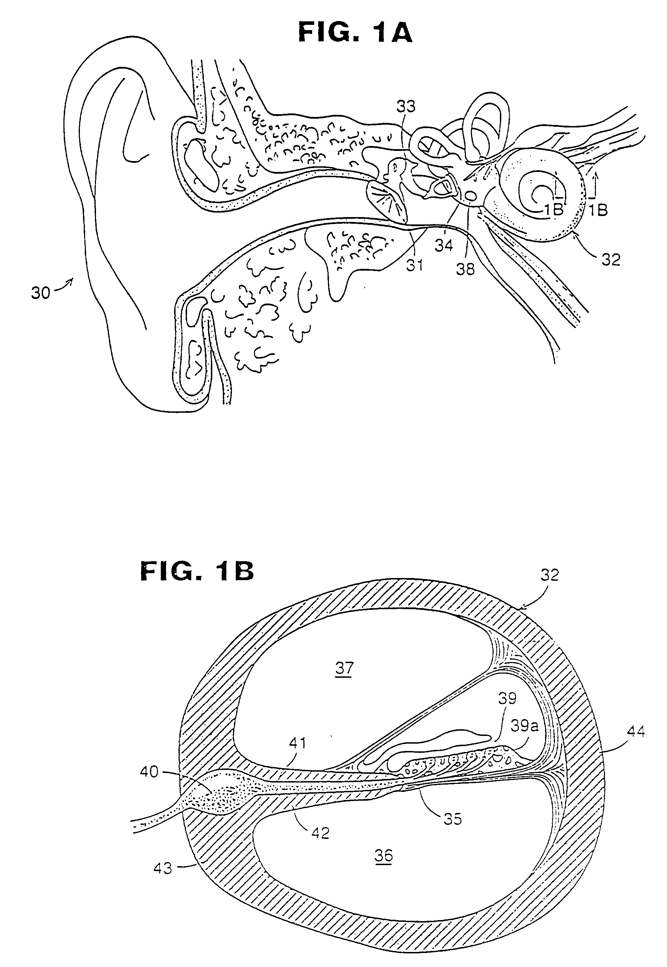

Generally speaking, the portions and structural components of a human ear with which the present invention is most closely concerned, though well known to those skilled in the art, are illustrated diagrammatically (without being drawn precisely to scale) in FIGS. 1 A and 1 B of the hereto appended drawings.

In the human ear 30 (see FIG. 1A) of a normal hearing person, sound impinges on the eardrum 31 and is transmitted into the cochlea 32 via a system of bones 33 called the ossicles, which act as levers to provide amplification and acoustic impedance matching, to a piston or membrane 34 called the oval window. The cochlea 32 is a spirally wound tube, resembling a snail shell, which is about 35 mm long when unrolled and is divided along most of its whole length (see FIG. 1B) by a partition 35 called the basilar membrane. The lower chamber 36 of the cochlea is called the scala tympani, and the upper chamber 37 is called the scala vestibuli. The cochlea is filled with a fluid with a viscosity of about twice that of water. The scala tympani 36 is provided with another piston or membrane 38 called the round window (see FIG. 1A), which serves to take up the displacement of the fluid when the oval window 34 is moved.

When the oval window is acoustically driven via the ossicles 33 the basilar membrane 35 is correspondingly displaced and vibrated by the movement of fluid in the cochlea. The displacement of the basilar membrane stimulates the hair cells 39 which are situated in a special structure 39a on the basilar membrane. Movements of these hairs produce electrical discharges in fibres of the auditory nerve through the intermediary of the cells 40 of the spiral ganglion 41 which are located in the modiolus or modiolar wall 42 at the radially inner wall 43 of the cochlea.

Hearing loss, which may be due to many different causes, is generally of two types, conductive and sensorineural. Of these, conductive hearing loss occurs where the normal mechanical pathways for sound to reach the hair cells in the cochlea are impeded, for example, by damage to the ossicles. Conductive hearing loss may often be helped by use of conventional hearing aids, which amplify sound so that acoustic information does reach the cochlea and the hair cells. Some types of conductive hearing loss are also amenable to alleviation by surgical procedures.

In many people who are profoundly deaf, however, the reason for their deafness is sensorineural hearing loss. This type of hearing loss is due to the absence or the destruction of the hair cells in the cochlea which are needed to transduce acoustic signals into auditory nerve impulses. These people are unable to derive any benefit from conventional hearing aid systems, no matter how loud the acoustic stimulus is made, because their mechanisms for transducing sound energy into auditory nerve impulses have been damaged. Thus, in the absence of properly functioning hair cells, there is no way auditory nerve impulses can be generated directly from sounds. To overcome this problem, there have been developed numerous cochlear implant systems which seek to bypass the hair cells in the cochlea (the hair cells are located in the vicinity of the radially outer wall 44 of the cochlea) by presenting electrical stimulation to the auditory nerve fibres directly, leading to the perception of sound in the brain and an at least partial restoration of hearing function. The general common denominator in these systems has been the implantation, into the cochlea, of electrodes which are responsive to suitable external sources of electrical stimuli and which are intended to transmit those stimuli to the ganglion cells and thereby to the auditory nerve fibres.

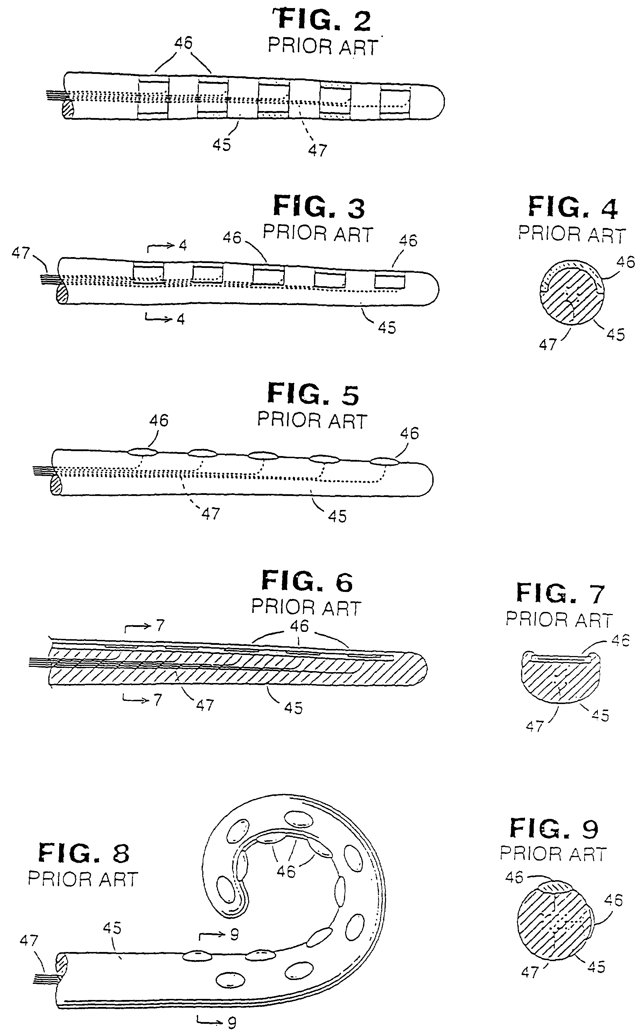

In the known cochlear implant systems, the carrier 45 for the stimulating electrodes 46, as shown in FIGS. 2-7, usually is a slightly tapered straight or minimally curved rod of a cylindrical or part-cylindrical cross-section and is made of a resiliently flexible biocompatible synthetic plastic material such as a silicone polymer available commercially under the name "Silastic". The electrode carrier is surgically placed into the scala tympani, in close proximity to the basilar membrane, and currents passed to the electrodes 46 via respective conductors or leads 47 embedded in the electrode carrier result in neural stimulation in proximate groups of ganglion cells. For this purpose, the electrodes or contacts, which are secured to the electrode carrier at spaced locations along its length, should preferably be located as close as possible to the modiolus, i.e., near the radially inner wall 43 of the cochlea, where the spiral ganglion cells 40 to be stimulated are located.

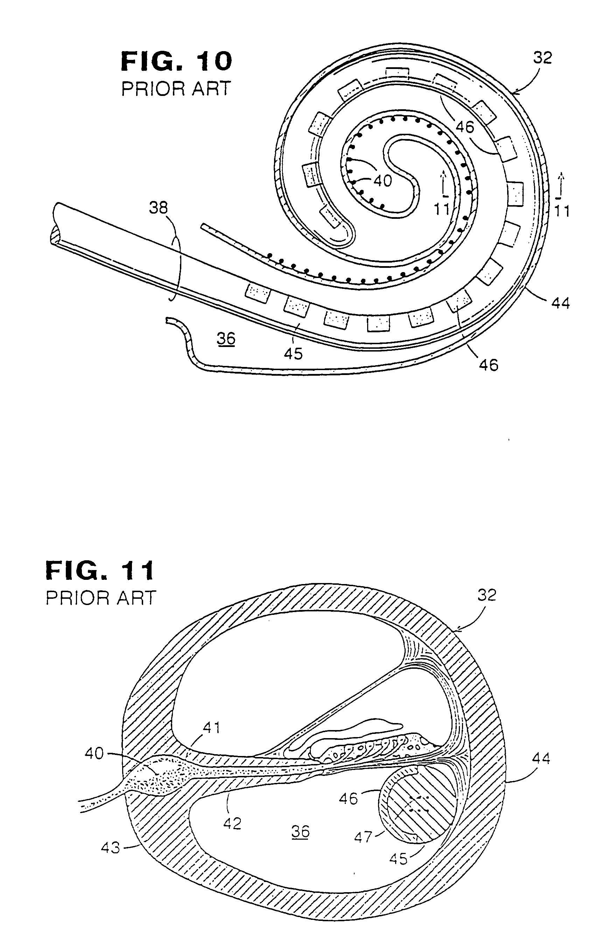

Here, however, a problem has been encountered. The electrode carriers, as already mentioned, are generally manufactured in a straight tapered rodlike form from a resilient polymer or like elastomeric material. By virtue of their being straight or only very slightly curved, the electrode carriers can be smoothly and easily inserted into the scala tympani 36 of the cochlea 32 through the opening in the round window 38 or through a small hole drilled into the basal part of the cochlea. When such an electrode carrier 45 (see FIG. 10) is so inserted into the cochlea 32, it flexibly curves into the spiral form of the scala tympani, but because the electrode carrier is resilient and has a memory tending to return it to its straight form, it ends up lying against, and closely following the curvature of, the radially outer wall 44 of the cochlea. As a result, the electrodes 46 on the carrier end up being located in the vicinity of the damaged and non-functional hair cells 39 on the basilar membrane 35 (see FIG. 11) but at a substantial distance (relatively speaking) from the inner wall 43 of the cochlea and hence also relatively far from the ganglion cells 40 in the modiolus, thereby limiting the achievable stimulation of the ganglion cells. In such a case, it is necessary to use stimulation currents which are somewhat higher than is usually deemed desirable, but that in turn leads to an undue overspread of the current and a reduction in the resolution of the stimulus.

In an attempt to overcome these drawbacks, which also have not been overcome by proposals to make the electrode carrier either in a more or less greatly curved form such as a spiral shape (see FIGS. 8 and 9) approximating the curvature of the cochlea and utilizing the memory of the carrier material to return the carrier to that shape after the carrier has been straightened for the purposes of the insertion operation, it was proposed in the aforesaid prior application to make the conventional straight rodlike electrode carrier in the form of two layers. Of these layers, the one which incorporates the electrical contacts or electrodes and their leads (herein designated the inner layer) was described as being made of a biocompatible silicone polymer, e.g., Silastic, which does not expand or swell when exposed to the water in the patient's body fluids, while the other layer (herein designated the outer layer) was described as being suitably adhered to the inner layer at the side of the latter directed away from the contact faces of the electrodes and as being made of a biocompatible silicone polymer (also Silastic) formulated through the addition of finely ground NaCl or polyacrylic acid or the like so as to have the property of expanding (swelling) under the action of the water in the patient's body fluids once the carrier has been inserted into the cochlea. By means of such an arrangement, it was suggested, the outer layer of the electrode carrier would, due to the liquid-generated expansion of the outer layer, be ultimately shifted away from its initial position at the radially outer wall of the cochlea to a second position within the scala tympani where the inner layer has its concavely curved surface, at which the contact faces of the electrodes are located, disposed in engagement with the radially inner wall of the cochlea and thereby in close proximity to the modiolus and the ganglion cells. As a solution to the indicated problem, however, this approach was not perfect because, even at best, achieving a post-implantation precise control of the expansion of the outer layer and of the resultant curvature of the inner layer of the electrode carrier is extremely difficult. Moreover, if an unduly great deviation from the desired curvature were to occur, this might necessitate corrective action by means of additional invasive surgery.

In EP-A-0002068, on which the preamble of independent claim 33 is based, there is disclosed a device and method for inserting an electrode carrier into a patient's cochlea. Use is made of the carrier's inherent resilience to ensure proper contact with the auditory receptors in the cochlea. However, this results in a carrier which is difficult to insert and may damage the walls of the cochlea. This is addressed by pre-stressing the carrier before insertion, to allow it to be inserted more easily, and then once positioned, removing the pre-stressing to allow the carrier to assume its natural shape to make good contact with the auditory receptors. This is a relatively complicated process and it is not always certain that the "natural curvature" of the unstressed carrier will make strong contact with the auditory receptors.

### Brief Description of the Invention

It is an object of the present invention, therefore, to provide a system for positioning a stimulating electrode carrier of a cochlear electrode implant assembly optimally in the cochlea of a human ear so as to avoid the drawbacks and disadvantages of known electrode positioning systems and instead to ensure the closest possible proximity of the electrodes to the modiolus and the ganglion cells.

It is another object of the present invention to provide a cochlear electrode positioning system which is characterized by the fact that, although the electrode carrier is initially straight so as to allow for an easy, gentle and controllable insertion thereof into the cochlea, the carrier when inserted is forced by the system to lie closely against and to closely follow the curvature of the radially inner wall of the cochlea, and by the fact that the system enables the curvature of the carrier to be automatically adjusted so as to match that of any size cochlea.

The present invention accordingly provides a cochlear electrode implant assembly which includes, in addition to the flexible rodlike initially straight electrode carrier, an electrode positioning system in the form of a flexible auxiliary positioning member which is likewise initially straight and is arranged in close juxtaposition to the electrode carrier longitudinally thereof on the side of the electrode carrier directed away from the modiolus-engaging contact faces of the electrodes and which, upon joint insertion thereof with the electrode carrier into the cochlea, assumes an enlarged outward curvature and presses over a major portion of its length against the radially outer wall of the cochlea so as to force the side of the electrode carrier where the contact faces of the electrodes are located as far as possible toward and into close proximity to the modiolus and hence into as close a proximity as possible to the ganglion cells.

Preferably the electrode positioning system includes means which serve to lock the assembly consisting of the electrode carrier and the auxiliary positioning member in place within the cochlea at the end of the insertion stage, thereby to firmly and stably secure the assembly against inadvertent movement in and withdrawal from the cochlea.

Preferably, the means for connecting the positioning member and the electrode carrier to each other are constructed so as to permit the positioning member to be withdrawn from the patient's ear easily and separately from and without disturbing the electrode carrier, thereby to facilitate a subsequent extraction of the electrode carrier if that should become desirable or necessary.

Generally speaking, in the implant assembly according to one aspect of the present invention the initially straight rod-like flexible electrode carrier, to which the multiple electrode elements or contacts (which may be as many as 22 or more in number) are secured in any desired way, is combined, as before, with an initially straight flexible positioning member which extends along the length of the electrode carrier and has a leading end region and a trailing end region connected to the leading end region and the trailing end region, respectively, of the electrode carrier in a manner to be more fully discussed hereinafter. In the assembly of the present invention, the electrode carrier and the positioning member are both preferably in the form of solid rods and are preferably made of respective biocompatible flexible plastic materials. Preferably, the electrode carrier is made of a flexible biocompatible silicone polymer such as Silastic. On the other hand, the positioning member preferably is made from a somewhat harder but still flexible biocompatible polymeric material such as nylon or Teflon™ (Teflon is a trademark for a material generically known as polytetrafluoroethylene or PTFE) or the like, which apart from having anti-friction properties also has a greater degree of stiffness and tensile strength than Silastic for enabling the positioning member to be used to pull the electrode carrier through the cochlea. The materials of which the electrode carrier and the positioning member in this embodiment of the present invention are made of must, of course, be such that they will not expand or swell upon being exposed to the water in the patient's body fluids.

The electrodes may be in the form of discrete layers or bands secured to and overlying respective full-circumference or part-circumference regions of the rod-shaped electrode carrier, or in the form of discrete either planar or domeshaped layers or other formations, arranged on a carrier of either circular or part-circular or similar cross-section, but in all cases located generally at that surface (hereinafter occasionally referred to as the lower surface) of the electrode carrier which, upon insertion of the carrier into the cochlea, will be concavely curved toward the modiolus.

In the assembly, the positioning member has its lower surface disposed adjacent that surface (the upper surface) of the electrode carrier which, upon insertion of the carrier into the cochlea, will be convexly curved away from the modiolus.

In the preferred embodiment of the present invention, the means for connecting the leading end region of the positioning member to the leading end region of the electrode carrier is a latchless rod-and-socket type of joint, while the means for connecting the trailing end region of the positioning member to the trailing end region of the electrode carrier is a ring-shaped member or band which is made of Silastic or the like and formed to be devoid of any sharp cutting edges. The ring defines a single interior passageway through which both the positioning member and the electrode carrier jointly extend with a close fit. The arrangement, which will be more fully described presently, is such that prior to the assembly (composed of the electrode carrier and the positioning member) being inserted into the scala tympani of the cochlea, both the electrode carrier and the positioning member are in their starting straight and substantially parallel state, with the medial region of the positioning member between its leading and trailing end regions being separated from (in the sense of being unconnected to) the juxtaposed medial region of the electrode carrier.

The insertion of the assembly into the patient's ear is preferably effected by the surgeon gripping and pushing the positioning member forwardly with the aid of a suitable insertion tool. That action, by virtue of the interconnected leading end regions of the electrode carrier and the positioning member, causes the electrode carrier to be effectively pulled into the cochlea. When the assembly reaches and enters into the spiral curvature section of the cochlea, the positioning member comes with its upper or outwardly directed surface up against and rides along the radially outer wall of the cochlea within the scala tympani, while concurrently therewith the electrode carrier comes with a medial section of its electrode-bearing lower or inwardly directed surface up against the proximate initial region of the radially inner or modiolar wall of the cochlea.

Thereupon, the continuing pushing force being exerted by the surgeon on the positioning member causes the trailing end region of the latter to advance somewhat relative to the trailing end region of the electrode carrier past the location of the connection between the two trailing end regions, as the forward motion of the electrode carrier is retarded somewhat due to the frictional drag exerted by the modiolar wall on the electrode carrier. By virtue of the interconnections of the leading and trailing end regions of the electrode carrier with the leading and trailing end regions of the positioning member, this advance of the positioning member relative to the electrode carrier results on the one hand in the medial region of the positioning member between its leading and trailing end regions assuming an outwardly bowed or arched configuration relative to the medial region of the electrode carrier and on the other hand in the leading end region of the positioning member curving away from the outer wall and toward the radially inner wall of the cochlea. The tip of the electrode carrier thus is forced across the width of the scala tympani until the leading end region as well as substantially the entire length of the electrode-bearing surface of the electrode carrier up to the trailing end region thereof is in close hugging contact with the modiolar wall. Upon completion of the insertion, therefore, the electrodes supported by the electrode carrier are then held by the positioning member, due to the latter being outwardly bowed or arched relative to the electrode carrier against the constraint of the radially outer wall of the cochlea, in as close a juxtaposition to the ganglion cells as possible.

Once the electrode carrier has been fully advanced in this manner to the desired final arched state thereof, the entire assembly remains firmly in place, because in the absence of a rearwardly directed pulling force exerted on the positioning member, the arched portion of the positioning member, despite its being made of a material with antifriction properties, is frictionally restrained by the ring member against reverse movement through the latter and hence is unable to straighten out. The positioning member thus remains in full surface contact over a major portion of its length with the radially outer wall of the cochlea. This stabilizes the assembly in the scala tympani and makes any inadvertent movement of the electrode carrier in the cochlea and especially a reverse movement thereof out of the cochlea effectively impossible.

The two parts of the assembly in this embodiment of the invention are connected to one another at their respective leading ends, as previously mentioned, by a latchless rod-and-socket type of joint, of which the rod-part is preferably supported by the positioning member and the correspondingly configured socket-part is supported by the electrode carrier. In the currently contemplated best mode of implementing the joint, the socket-part thereof is constituted by a small tubular wire coil formation preferably utilizing a bundle of about 2-4 insulated wires which are identical to the conductors for the electrodes and are embedded in the electrode carrier at the time of its manufacture but are not connected to the signal source. Alternatively, of course, the coil formation can be made of a single somewhat thicker wire. The tubular wire coil formation is embedded in the tip end region of the electrode carrier so that the axial hollow interior of the coil slants forwardly somewhat in the direction from the upper surface of the electrode carrier toward the lower surface thereof, with the interior of the coil being accessible through an appropriate opening provided in the upper surface of the electrode carrier in axial alignment with the coil formation.

Correspondingly, the rod-part of the joint is constituted by the leading end region of a single guide wire running longitudinally through the positioning member. The said leading end region of this wire is of conventionally round cross-section, has a diameter of about 0.1 mm and a length of about 2 mm, and protrudes obliquely downwardly from the tip of the positioning member so as to be adapted to be slidably received in the hollow interior of the coil formation within the electrode carrier. The remainder of the guide-wire is preferably flattened into a generally rectangular cross-section, has a thickness of about 0.03 mm and a width of approximately one half of the width of the positioning member, and extends through the entire length of the positioning member. The guide wire has a flat face thereof facing toward the electrode carrier, so that the guide wire serves as a stiffening element which renders the positioning member resistant to side to side flexure in the plane of the guide wire while permitting essentially unhindered flexure of the positioning member in a direction perpendicular to that plane, i.e., in the direction toward and away from the electrode carrier.

According to another embodiment, the two parts of the assembly are linked or articulated to one another at their respective leading ends by a ball-and-socket type of joint, of which the ball-part (which may have either a spherical or a non-spherical shape) preferably is supported by the electrode carrier and the correspondingly configured socket-part is formed on the positioning member. In the currently contemplated best mode of implementing the linkage, a small number of wires (preferably 2-4 insulated wires identical to the conductors for the electrodes but not connected to the signal source) are run through the electrode carrier at the time of its manufacture, and these wires terminate outside the tip of the electrode carrier. At the distal ends of these wires a suitable distance from the tip of the electrode carrier, the insulation is stripped off the wires and the so exposed end regions thereof are melted together to form a ball (or a functionally like solid body), so that the latter is effectively supported by the electrode carrier through the intermediary of the wires. Correspondingly, the leading end region of the positioning member is provided with a frontwardly open axial slit disposed in the common axial plane of the assembly and dimensioned, both lengthwise and widthwise, to accommodate the portion of the ball-supporting bundle of wires between the ball and the tip of the electrode carrier. The positioning member is also provided at the rearwardmost end of the slit on the outer surface of the positioning member with an enlarged suitably configured open-topped recess or depression facing away from the electrode carrier to constitute the socket for receiving the ball.

The currently preferred means for ensuring that the assembly according to the present invention remains locked in place in any given adjusted state of the assembly in the patient's ear is a fitting having a body made of a biocompatible plastic material such as Silastic and having the form of a ring or band of a generally D-shaped cross-sectional configuration, with the flat wall of the D defining the inner circumferential boundary surface of the ring. The inner configuration of the ring closely matches the combined outer configuration of the assembled electrode carrier and positioning member. Thus, when the positioning member and the electrode carrier are in their properly assembled state, the ring closely surrounds and embraces them at their trailing end regions and there connects them to each other. It will be understood that although the positioning member is made of a material (PTFE or the like) having antifriction properties, in the absence of any longitudinal force applied by the surgeon to the positioning member the Silastic ring frictionally restrains the positioning member against any free or unforced longitudinal displacement through the ring and relative to the electrode carrier.

On the other hand, the antifriction property of the material of which the positioning member is made ensures that when a longitudinal force is applied by the surgeon to the positioning member1 the latter can slide through the ring and relative to the electrode carrier. Thus, when the surgeon, while the leading end regions of the positioning member and the electrode carrier are connected to each other by the interfitted parts of the latchless rod-and-socket joint, exerts (whether by hand or with the aid of a conventional insertion tool) a sufficient longitudinal forwardly directed pushing force on the positioning member rearwardly of the location where the trailing end regions of the positioning member and the electrode carrier are connected to each other by the ring, the positioning member is able to slide through the ring. As a result thereof, the portion of the positioning member located between the two connections, i.e., the portion between the ring and the rod-and-socket joint, assumes an outwardly arched or bowed configuration relative to the upper surface of the electrode carrier, so that by virtue of the upper surface of the positioning member coming into engagement with the radially outer wall of the cochlea, the electrode carrier is displaced across the width of the scala tympani so as to bring the contact elements on the lower surface of the electrode carrier into the closest possible proximity to the modiolus and the ganglion cells. Likewise as a result of the arching of the positioning member, the oblique orientation, relative to the axis of the ring, of that portion of the positioning member which is proximate to the ring in the region between the two connecting means, aided by the frictional contact between the ring and both the positioning member and the electrode carrier, ensures that the positioning member cannot of its own accord slide rearwardly through the ring and that the entire assembly will remain stable in its inserted position within the cochlea.

The antifriction character of PTFE also comes into play when the implanted electrode assembly is to be extracted from the patient's ear. Should that become necessary for any reason, the surgeon, while holding the ring stationary in the patient's outer ear, will first exert on the positioning member a rearwardly directed longitudinal pulling force of sufficient magnitude to overcome the frictional interaction between the ring and the positioning member. This will cause a rearward sliding of the positioning member through the ring accompanied initially by a straightening of the arched portion of the positioning member while the electrode carrier remains in place. Ultimately, the rod-part of the joint between the leading end regions of the positioning member and the electrode carrier, which is not latched or locked to the wire coil formation in any way, will be pulled slidingly out of the associated socket-part of the joint to permit the positioning member to be fully withdrawn from the cochlea. What happens to the electrode carrier after such an extraction of the positioning member is dependent on whether or not scar tissue will have grown in the cochlea and encapsulated the electrode carrier. If encapsulation has taken place, the electrode carrier will be held in place by the scar tissue that has grown around it and will remain in its operational adjusted position, with the electrodes or contact elements in close proximity to the modiolus. If no encapsulation has taken place, the "memory' of the material of which the electrode carrier is made will cause the latter to try to return to its original straight form, which will leave the electrode carrier partially uncoiled and with its upper surface bearing against the radially outer wall of the cochlea. In either condition, of course, the electrode carrier can be relatively easily extracted from the cochlea.

An alternative means according to the invention for locking the assembly in place is a fitting having a body in the form of a generally S-shaped structure made of a strip of titanium or like biocompatible metal and defining in its opposite end regions (the loops of the NS") a pair of substantially parallel passageways extending through the fitting parallel to its axis and separated from each other by a medial axial partition (the centre web of the Us"), the passageways being intended, one for accommodating the electrode carrier and the other for accommodating the positioning member. The portion of the body of the fitting which defines the passageway through which the electrode carrier extends is fixed to the trailing end region of the electrode carrier, e.g., by being crimped thereonto, somewhat rearwardly of the trailing end of the array of electrodes, while the portion of the body of the fitting which defines the passageway through which the positioning member extends is provided with wedgelike means arranged to permit forward movement but prevent reverse movement of the positioning member through that passageway. The wedgelike means preferably has the form of a short strip of metal which is affixed at one end edge thereof to the medial partition of the fitting and extends from its attachment location along the proximate face of the partition in the direction of forward movement of the positioning member, the wedging strip terminating at an opposite relatively sharp free end edge thereof and being oriented at an inclination relative to the partition and toward the axis of the passageway through which the positioning member extends.

It will be understood, therefore, that the positioning member can slide freely past the free end edge of the wedging strip when moving in the forward direction during the insertion stage, i.e., while the pushing force is being exerted on the positioning member. On the other hand, upon completion of the insertion stage (or, for that matter, at any time during the insertion stage), if the pushing force is relaxed and the positioning member starts to move in the reverse direction, the free end edge of the wedging strip will immediately bite, i.e., penetrate slightly on a bias, into the positioning member and will create a pawl-like wedging effect, thereby to inhibit any further reverse movement of the positioning member and to lock the entire implant assembly in place.

In another embodiment of the invention, the positioning member again has a straight rodlike configuration in the starting (pre-insertion) state of the assembly and is located lengthwise of and in close parallel relation to the electrode carrier. In this case, however, the positioning member is not a solid rod but rather is constituted by an elastic axially compressed tube of Silastic or like biocompatible plastic material, this tube even in its compressed state being longer than the array of electrodes on the electrode carrier. A platinum wire (this is preferred, although any type of tension member of a biocompatible material other than platinum or platinum alloy, even of a non-metallic material, can be used as long as it has the requisite physical properties, and the term wires should be so interpreted) extends axially slidably through the tube to impart stiffness to the tube. The leading end region of the wire, which protrudes from the leading end of the compressed tube, has a reverse bend formed therein, defining a hooklike portion the free end of which is secured to, e.g., molded into, the tip of the electrode carrier, with the bend in the wire constituting a hinge portion about which the positioning member can move angularly (pivotally) relative to the electrode carrier in the common axial plane of the assembly. The compressed tube has its leading end region located adjacent the leading end region of the electrode carrier and has its trailing end region fixedly secured or anchored to the electrode carrier somewhat rearwardly of the trailing end of the electrode array. The trailing end region of the wire extends somewhat beyond the trailing end region of the compressed tube and is initially straight and held fast, e.g., clamped, to the electrode carrier at that location. In this way, the wire keeps the compressed tube straight as well and prevents any premature movement of the positioning member relative to the electrode carrier.

As before, the initial insertion of the assembly into the cochlea takes place while both the positioning member and the electrode carrier are in their straight state, with the surgeon in this case exerting the needed pushing force on the electrode carrier. During the entire insertion operation, the trailing end of the wire remains clamped in place on the electrode carrier, and thus the wire is constrained against movement relative to the carrier, thereby ensuring that the compressed tube also remains straight. Thus, when the assembly consisting of the positioning member and the electrode carrier enters the spirally curved section of the cochlea, both the positioning member and the electrode carrier become correspondingly curved, with the positioning member over substantially its entire length being in contact with the radially outer wall of the cochlea, and with the electrode carrier closely following the curvature of the positioning member and consequently disposing the contact faces of the electrodes out of the desired degree of proximity to the modiolar wall and the ganglion cells.

Once the insertion operation has been carried out to the fullest extent possible, however, the trailing end of the wire is unclamped from the electrode carrier. This releases the constraint exerted by the wire on the compressed tube and permits the tube to expand longitudinally so as to assume, by virtue of the fact that the leading and trailing end regions of the tube are effectively held stationary on the electrode carrier and that an additional portion of the new released wire is drawn into the tube, an outwardly arched or bowed configuration relative to the electrode carrier. As a result, the expanding tube acts in the manner of a spring and, aided by the stiffening effect of the wire confined within the tube, exerts an outward force on the radially outer wall of the cochlea. By virtue of the interconnection of the leading end regions of the positioning member and the electrode carrier, therefore, the arching of the positioning member forces the tip of the electrode carrier across the width of the scala tympani until it comes into contact with the radially inner wall of the cochlea and concomitantly therewith causes the electrode-bearing surface of the electrode carrier to come along its entire length into close hugging contact with the modiolar wall. It will furthermore be understood, in this regard, that when, in the described final position of the assembly, the trailing end region of the wire is again fastened, i.e., reclamped, to the electrode carrier, the arched positioning member constituted by the tube and the wire which is enclosed therein constitutes the means for locking the assembly in place by virtue of the fact that, in the absence of a pulling force exerted on the wire, the arched positioning member is effectively precluded from returning to its original straight state, so that any instability or inadvertent movement of the assembly in the cochlea or reversely out of the cochlea is effectively inhibited.

In accordance with yet another embodiment of the invention, the assembly initially consists of a straight rodlike electrode carrier which is made of Silastic silicone plastic material that is inert to and does not expand under the action of the water in the patient's body fluids, and a straight rodlike positioning member which is likewise made of Silastic silicone plastic material but one that is formulated to be responsive to and to expand or swell under the action of the water in the patient's body fluids. As disclosed in the aforesaid application Ser.No. 211,269, the Silastic silicone polymer may be rendered water-swellable by mixing it with predetermined amounts of finely ground NaCI, polyacrylic acid, or like substances which in effect draw in water when exposed to the body fluids.

The electrode carrier and the positioning member initially are effectively equal in length and are molded or otherwise secured, e.g., bonded, to each other at their respective leading and trailing end regions, and between those locations they are disposed substantially parallel to and separated from (unconnected to) each other.

In use, this assembly is initially inserted into the scala tympani, as before, while still in its straight state, and the insertion is then continued by the surgeon pushing the assembly to advance along the spirally curved section of the cochlea. During this stage of the operation, the positioning member and the electrode carrier both become correspondingly curved, with the positioning member over substantially its entire length being in contact with the radially outer wall of the cochlea, and with the electrode carrier closely following the curvature of the positioning member and disposing the contact faces of the electrodes out of the desired close proximity to the modiolus. Once the insertion operation has been carried out to the fullest possible extent, however, no further action by the surgeon to shift the electrode carrier to its desired position is required. Rather, the subsequent swelling of the positioning member 'over a period of time as it is exposed to the patient's body fluids will cause the positioning member to assume, by virtue of its fixed connection at both ends to the electrode carrier, an outwardly bowed or arched configuration relative to the electrode carrier so as to exert an outward force against the radially outer wall of the cochlea. As a result of the expansion of the positioning member, therefore, the electrode carrier is forced by the positioning member, due to the interconnection between the leading and trailing end regions of the latter with the leading and trailing end regions of the electrode carrier, toward and against the radially inner wall of the cochlea, which causes the electrode-bearing surface of the electrode carrier to come along its entire length into close hugging contact with the modiolar wall. Here too, the arched positioning member, which in the stated environment is precluded from returning to its original straight state, serves as the means for locking the assembly in place and for preventing any instability of the assembly in or reverse movement thereof out of the cochlea.

The principal advantage of the positioning system according to all of the embodiments of the present invention is that the presence of the positioning member alongside the electrode carrier, with their opposite end regions connected to and with their medial regions separated from each other, generally ensures that the electrode-bearing face of the electrode carrier ends up in close contact with the modiolus so as to juxtapose the electrode contact faces as close to the ganglion cells as possible. It will be appreciated that anatomical abnormalities may make this less effective in some cases, however, in all cases the electrode carrier will tend to move towards the modiolus. A related advantage is that the curvature of the electrode carrier is automatically adjusted to that of the cochlea irrespective of the size of the cochlea, so that a pre-insertion adaptation of the system to any particular patient's cochlea is not required.

### Brief Description of the Drawings

The foregoing and other objects, characteristics and advantages of the present invention will be more clearly understood from the following detailed description thereof when read in conjunction with the accompanying drawings, which are basically schematic or diagrammatic in nature and should be viewed as such, in which:
FIGS. 1A and 1B, which have already been discussed previously herein, are diagrammatic interior views, not necessarily drawn to scale, of those portions of the human ear, including the cochlea, the structural and functional characteristics of which are relevant to an understanding of the present invention, with FIG. 1A generally showing the overall structure of the ear partly in elevation and partly in longitudinal section, while FIG. 1 B is a greatly enlarged cross-sectional view, taken along: the line 1B-1B in FIG. 1A, which generally illustrates the structure of the cochlea;
FIGS. 2-9, which have also been discussed above, are schematic illustrations of various types of known electrode carriers for cochlear implant systems, with FIGS. 2, 3, 5, 6 and 8 being fragmentary elevational views, partly in section, of the electrode carriers, while FIGS. 4, 7 and 9 are cross-sectional views taken along the lines 4-4, 7-7 and 9-9 in FIGS. 3, 6 and 8, respectively;
FIGS. 10 and 11, which have already been discussed above, schematically illustrate the cochlea of a human ear having one of the known electrode carriers inserted thereinto and show the unsatisfactory final position of the electrode carrier and its array of electrodes in the cochlea, with FIG. 11 being a greatly enlarged cross-sectional view taken along the line 11-11 in FIG. 10;

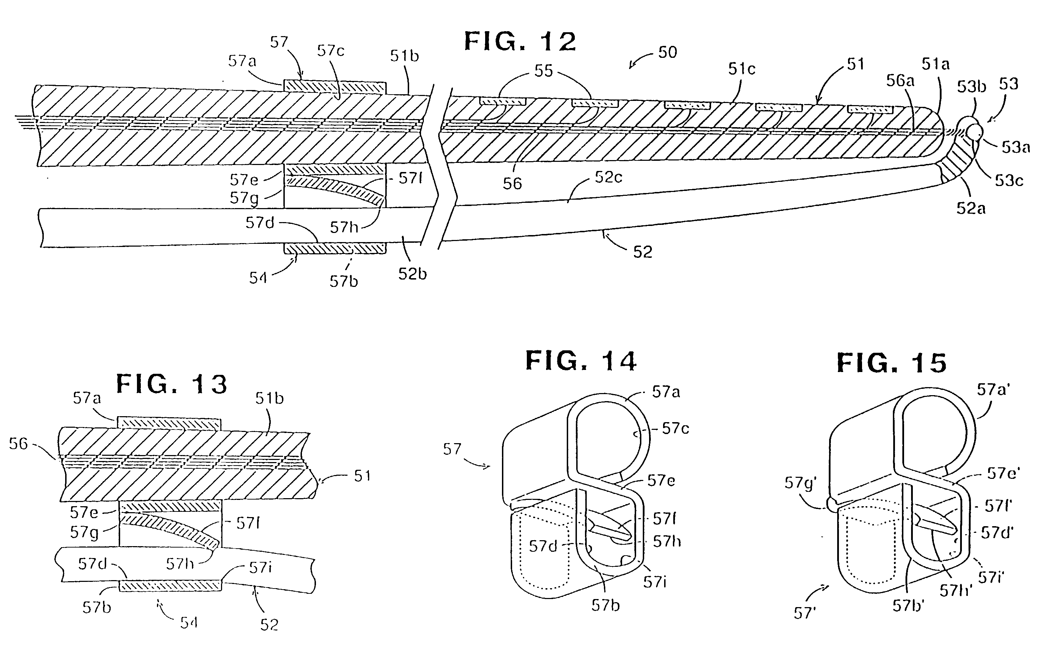

FIG. 12 is a fragmentary longitudinal sectional view, partly in elevation, of a cochlear electrode carrier positioning member assembly according to one embodiment of the present invention in the pre-insertion state of the assembly and illustrates one type of joint for interconnecting the leading end regions of the electrode carrier and the positioning member and a wedging type of fitting for interconnecting the trailing end regions of the electrode carrier and the positioning member for one-directional movement of the latter relative to the former;
FIG. 13 is a view similar to FIG. 12 but illustrates the assembly in its post-insertion state and shows the fitting as wedging the positioning member against reverse movement for locking the assembly in place in the cochlea;
FIG. 14 is a perspective illustration of the wedge-type fitting shown in FIGS. 12 and 13;
FIG. 15 is a perspective illustration of a slightly modified version of the fitting shown in FIG. 14;

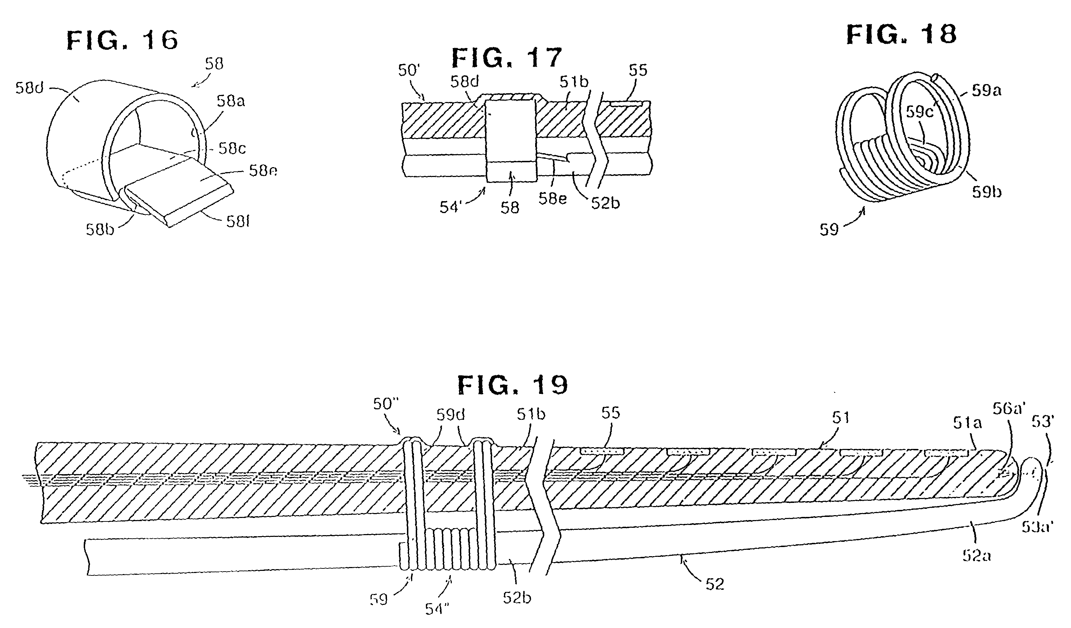

FIG. 16 is a perspective illustration of a different form of wedge-type fitting which has a generally tubular structure and may be used with the cochlear electrode implant assembly of FIGS. 12 and 13;
FIG. 17 is a fragmentary side elevational view, partly in section, of a cochlear electrode implant assembly utilizing the wedge-type fitting of FIG. 16 and shows the fitting as having its electrode carrier-accommodating section molded to the electrode carrier and as having its positioning member-accommodating 'section in the wedging state thereof;
FIG. 18 is a perspective illustration of yet another form of tubular fitting (59) which may be used with the cochlear electrode implant assembly of FIGS. 12 and 13 but permits bidirectional movement of the positioning member and therefore requires a crimping of a portion of the fitting onto the positioning member to lock the latter against reverse movement;
FIG. 19 is a fragmentary side elevational view, partly in section, of a cochlear electrode implant assembly utilizing the crimp-type fitting of FIG. 18 and shows the fitting as having its electrode carrier-accommodating section molded to the electrode carrier;

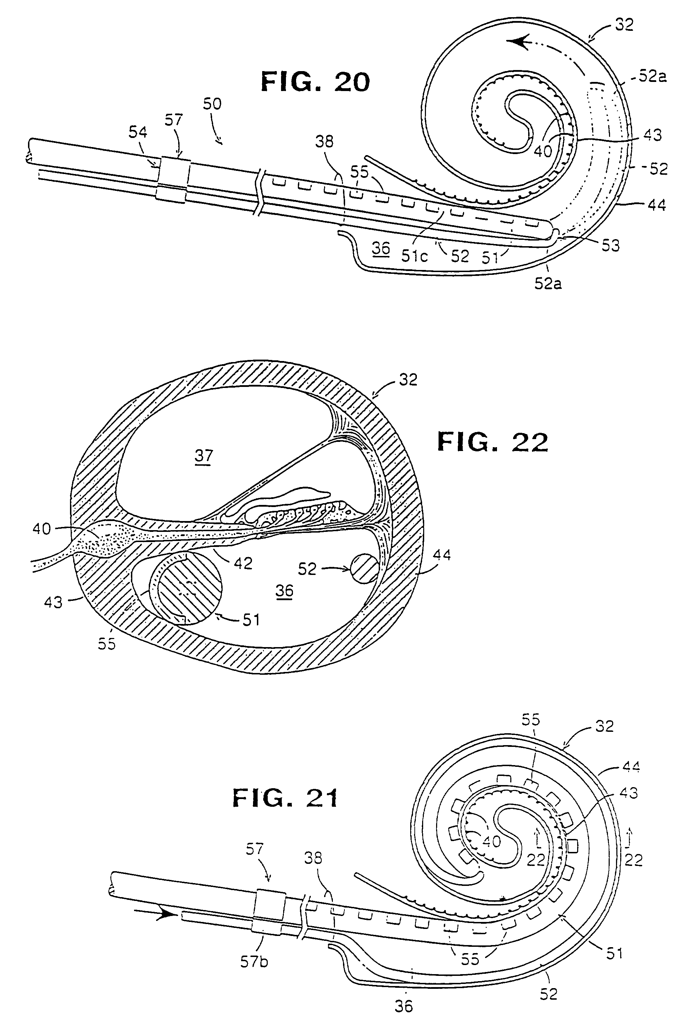

FIGS. 20 and 21 are schematic illustrations in longitudinal section, respectively, of the initial and final stages of the insertion of a cochlear electrode carrier and positioning member assembly according to the embodiment of the invention shown in FIGS. 12 and 13 into the cochlea of a human ear, with FIG. 21 showing the satisfactory final locked position of the electrode carrier;
FIG. 22 is a greatly enlarged cross-sectional view taken along the line 22-22 in FIG. 21;

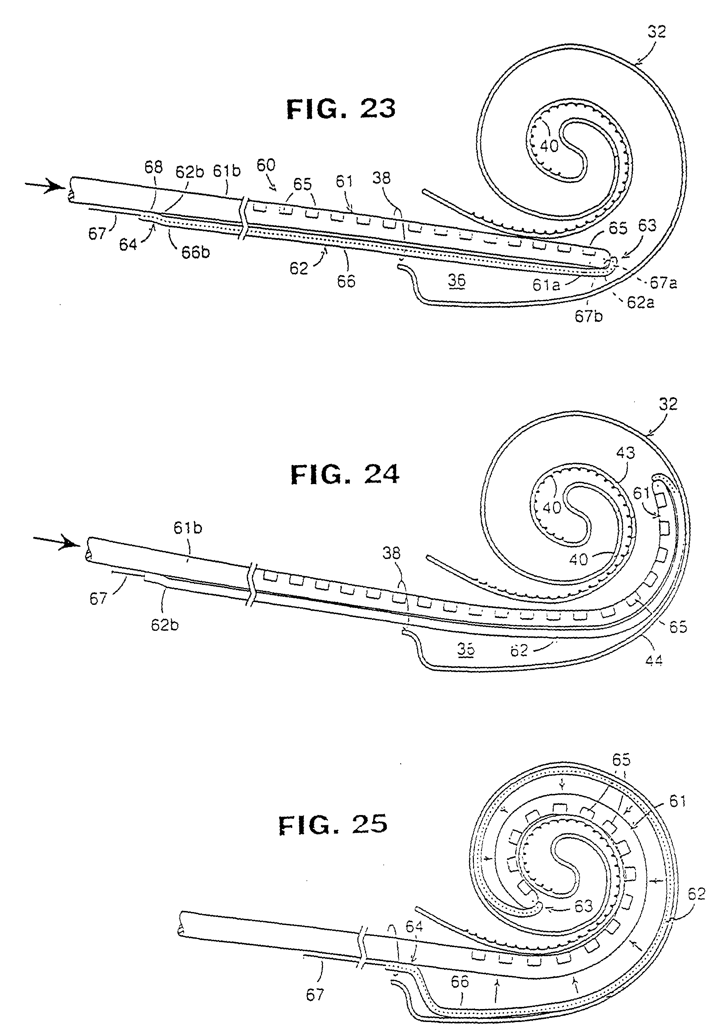

FIGS. 23, 24 and 25 are schematic illustrations in longitudinal section, respectively, of the initial, intermediate and final stages of the insertion of a cochlear electrode carrier and positioning member assembly according to a second embodiment of the present invention into the cochlea of a human ear, with FIG. 25 showing the satisfactory final locked position of the electrode carrier;

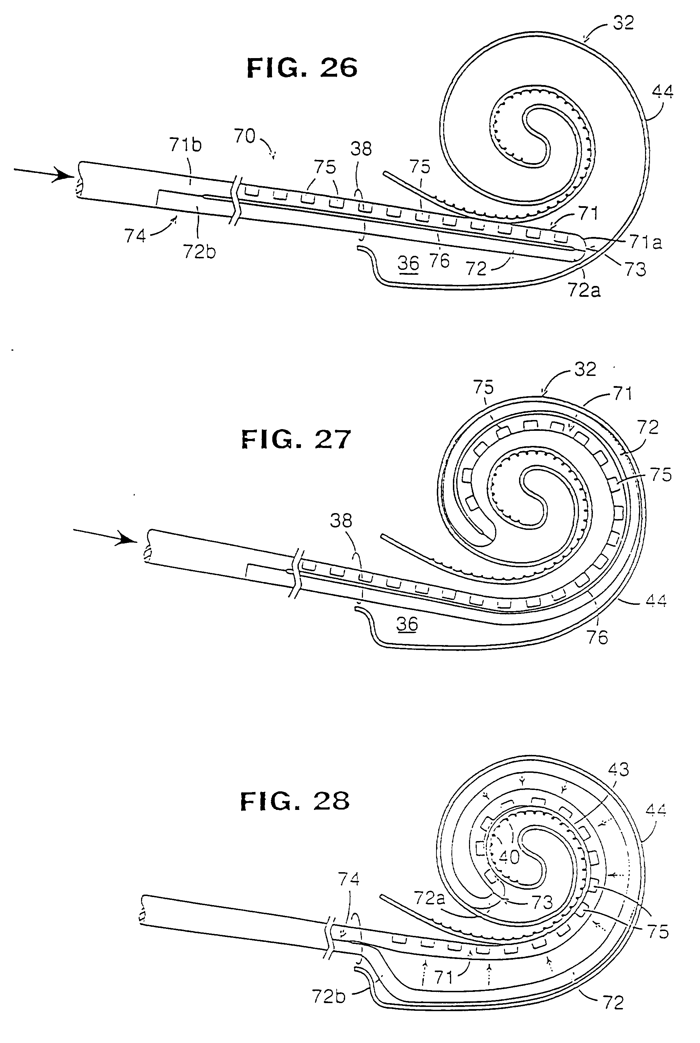

FIGS. 26, 27 and 28 are schematic illustrations in longitudinal section, respectively, of the initial, intermediate and final stages of the insertion of a cochlear electrode carrier and positioning member assembly according to a third embodiment of the present invention into the cochlea of a human ear, with FIG. 28 showing the satisfactory final locked position of the electrode carrier;

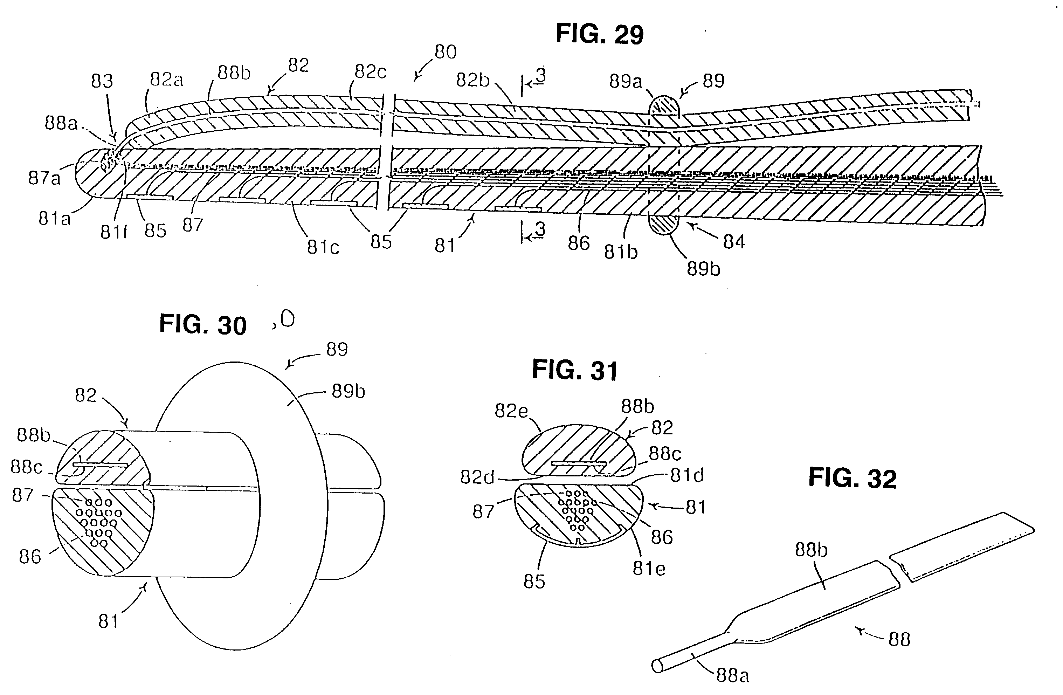

FIG. 29 is a fragmentary longitudinal sectional view of a cochlear electrode carrier/positioning member assembly according to a fourth embodiment of the present invention essentially in the preinsertion state of the assembly and illustrates a latchless rod-and-socket joint for interconnecting the leading end regions of the electrode carrier and the positioning member and a ring-shaped member for interconnecting the trailing end regions of the electrode carrier and the positioning member;
FIG. 30 is a perspective illustration, drawn to an enlarged scale, of the portion of the assembly of FIG. 29 in the region of the interconnection between the trailing end regions of the positioning member and the electrode carrier by the ring-shaped member;
FIG. 31 is a sectional view taken along the line 3-3 in FIG. 29; FIG. 32 is a perspective illustration of the guide wire for the positioning member of the assembly and shows the guide wire as having a cross-sectionally round front end portion to constitute the rod-part of the joint between the leading end regions of the positioning member and the electrode carrier and as having a generally planar or flat body portion for stiffening the positioning member;

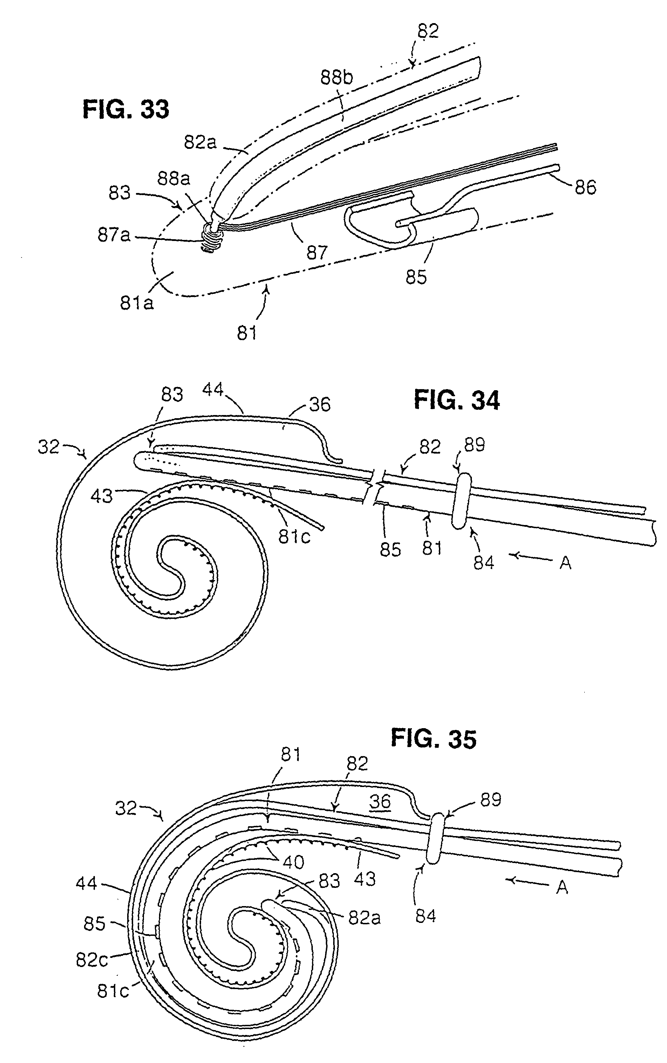

FIG. 33 is a perspective illustration, drawn to a somewhat enlarged scale, of the leading end region of the cochlear electrode implant assembly of FIG. 29;

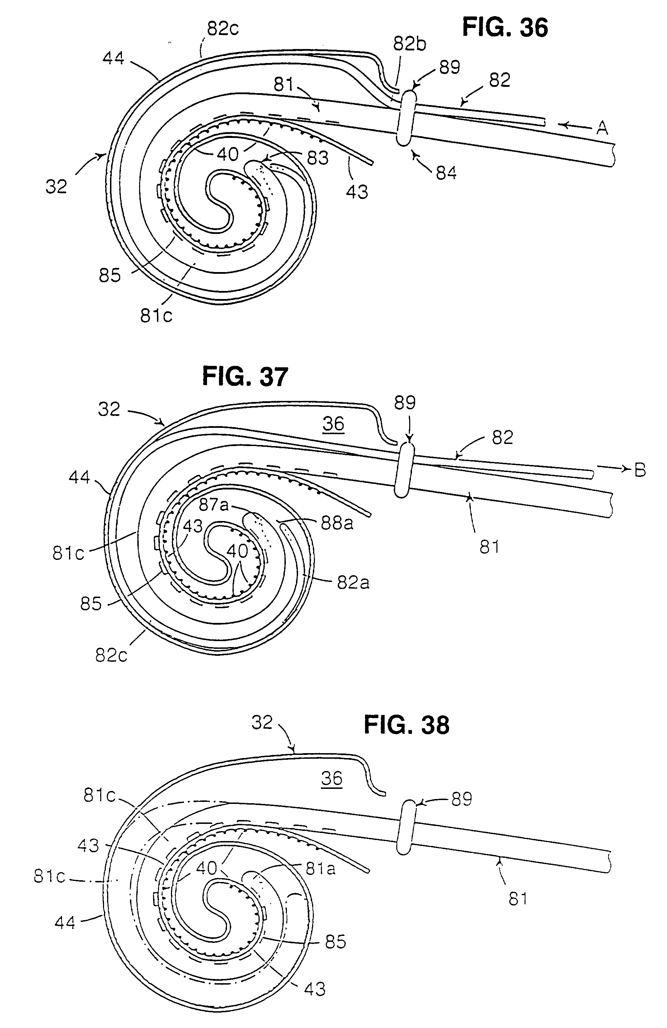

FIGS. 34, 35 and 36 are schematic illustrations, in longitudinal section, of, respectively, the initial, intermediate and final stages of the insertion of a cochlear electrode carrier and positioning member assembly according to the fourth embodiment of the present invention into the cochlea of a human ear, with FIG. 36 showing the satisfactory final locked position of the electrode carrier;
FIG. 37 is a schematic illustration, in longitudinal section, of the initial stage of the extraction of the positioning member according to the fourth embodiment from the patient's ear; and
FIG. 38 is a similar view to FIG. 38 showing two possible positions of the electrode carrier after separation of the positioning member therefrom and preparatory to an extraction of the electrode carrier from the ear.

### Detailed Description of the Invention

### First Embodiment

Referring now to the drawings in greater detail, FIGS. 12 and 13 show a cochlear electrode implant assembly 50 which, in accordance with a first embodiment of the present invention, includes an electrode carrier 51 of a per se known type and a novel associated auxiliary positioning member 52, together with means 53 for connecting the leading end region 51 a of the electrode carrier to the leading end region 52a of the positioning member and with means 54 for connecting the trailing end region 51b of the electrode carrier to the trailing end region 52b of the positioning member. Thus, on the one hand the electrode carrier 51 is shown, merely by way of example, as having the structure of the electrode carrier of FIGS. 3 and 4, i.e., as being a straight flexible solid rod of circular cross-section, which is made of Silastic silicone plastic or an equivalent biocompatible material, which at one side region thereof and in a medial region 51c between the leading and trailing end regions 51a and 51b bears an array of longitudinally spaced electrode elements 55 in the form of part-cylindrical bands or layers of a biocompatible metal such as platinum or platinum alloy, and which further has a series of insulated electrical conductors 56 made of like biocompatible metals and embedded in its interior for conducting stimuli in the form of electrical sound-representing signals or impulses to the various electrode elements. (It will be understood, of course, that the electrode carrier and its electrode elements may just as well have any of the other configurations thereof shown in FIGS. 2 and 5-9, although not the precurved spiral shape shown in FIG. 8) On the other hand, the auxiliary positioning member 52 in this embodiment of the invention is a flexible solid rod of preferably circular cross-section, which is shown as being somewhat thinner than the electrode carrier 51 (although this is not an essential or indispensable condition). The positioning member, which initially is also generally straight, is juxtaposed to the side region of the electrode carrier opposite to the one where the contact faces of the electrode elements 55 are exposed and extends lengthwise of and along the electrode carrier so as to have its leading and trailing end regions 52a and 52b adjacent, respectively, to the leading and trailing end regions 51a and 51b of the electrode carrier and so as to have its medial region 52c between the leading and trailing end regions 52a and 52b separated from, i.e., unconnected to, the medial region 51c of the electrode carrier. The positioning member is likewise made of a biocompatible material which may be Silastic silicone plastic but, for purposes of enhanced stiffness and tensile strength, is preferably a harder material such as nylon, Teflon™ or a Teflon™-like material (polytetrafluoroethylene), or the like.

The connection 53 between the leading end region 52a of the positioning member and the leading end region 51a of the electrode carrier in the embodiment of FIGS. 12 and 13 is effected by means of a ball-and-socket joint which, in the illustrated arrangement, includes a ball element supported by the electrode carrier and a socket portion located on the positioning member. More particularly, the electrode carrier 51 at the time of its manufacture is provided in its interior with a few, preferably from 2 to 4, additional insulated wires 56a which are physically identical to and extend longitudinally through the electrode carrier like the wires 56 but are not connected to the source of the electrical stimuli. The bundled wires 56a protrude a short distance forwardly beyond the tip of the electrode carrier and at their distal end regions are melted and fused to each other into the form of a ball 53a. Correspondingly, the positioning member 52 is provided at its tip with a frontwardly open and rearwardly extending axial slit 53b just wide enough to accommodate the thickness of the bundle of wires 56a which support the ball element 53a, and at its side facing away from the electrode carrier the positioning member is provided with an appropriately configured and dimensioned open-topped arcuate recess or depression 53c which symmetrically bridges the rearwardmost end of the slit. During the assembly operation, therefore, it is merely necessary to slip the projecting section of the bundle of wires into the slit and to slide the ball over the top surface of the positioning member until it is received in the recess.

It should be understood, in this regard, that although as a general proposition the ball element 53a of the ball-and-socket joint 53 will preferably be a ball having a spherical or hemispherical shape while the recess 53c constituting the socket portion of the joint will have a depth, curvature and overall contours enabling the ball to be properly and securely received and seated therein, other forms thereof could also serve the functional purposes of the joint; for example, the "ball element" could be cylindrical or rod like in shape adapted to be received and seated in a correspondingly shaped groove or notch. The terms "ball elements and "ball-and-socket joint" thus are intended to be, and should be, interpreted in their broadest possible sense.

The connection 54 between the trailing end region 51b of the electrode carrier and the trailing end region 52b of the positioning member in the embodiment of FIGS. 12 and 13 is effected by means of a metallic double tubular fitting (59) 57 which is made of titanium strip and has a body in the form of a generally S-shaped structure (see FIG. 14). The essentially tubular loops 57a and 57b of the SZ define a pair of substantially parallel passageways 57c and 57d which extend through the fitting along respective axes parallel to the axis of the fitting and are separated from each other by an interior partition 57e constituted by the center web of the S." Within the passageway 57d there is provided, in accordance with one version of the fitting 57 contemplated by the present invention, an additional strip 57f of titanium which along one end edge thereof is spot-welded to the partition 57e at 579 and extends forwardly from that location through the passageway 57d at an inclination toward the axis of that passageway. The distal end edge 57h of the strip 57f is relatively sharp for a purpose to be more fully explained presently.

The passageway 57c is initially dimensioned to freely slidably accommodate the electrode carrier 51 for insertion thereof into that passageway, with the portion of the titanium strip which constitutes the loop 57a being sufficiently flexible and strong to permit it to be crimped onto and thereby permanently affixed to the electrode carrier. The passageway 57d, on the other hand, is dimensioned so that, as is clear from FIG. 12, at all times a substantially free and unimpeded sliding movement of the positioning member 52 relative to the fitting 57 and the electrode carrier 51 fixed thereto can take place only in the forward direction through the gap defined in the passageway 57d between the bottom of the loop 57b and the distal end edge 57h of the inclined strip 57f and so that, as is clear from FIG. 13, no movement of the positioning member relative to the fitting in the reverse direction can take place because immediately upon commencement of such a movement the angled strip 57f will exert a pawl-like wedging action on the positioning member by virtue of the sharp end edge 57h of the strip biting into the proximate side region of the positioning member. That wedging action will be further assisted by the fitting, and especially so when the i positioning member has assumed its outwardly bowed or arched configuration (only the beginning portion of which is shown in FIG. 13), because concurrently with the edge 57h biting into one side region of the positioning member, the equally sharp inner bottom edge 57i of the loop 57b will bite into the opposite side region of the positioning member.

Referring now to FIG. 15, the S-shaped or double tubular fitting (59) 57' there shown is functionally fully and structurally almost fully identical to the fitting 57.

The only difference between them is that the fitting 57' is totally a one-piece rather than a two-piece structure. This is accomplished by utilizing a starting titanium strip which in its original flat state (not shown but readily visualizable from FIG. 15) is essentially T-shaped in outline, with the wedging strip 57f' being formed as a part of the overall shaping operation by bending the laterally projecting extension (the leg of the) of the middle portion of the original titanium strip which ultimately constitutes the partition 57e' over at 579' into the confines of the loop 57b' and the passageway 57d' defined therein.

The provision and use of a somewhat different form of wedging-type fitting 58 for effecting the connection 54' between the trailing end regions 51b of the electrode carrier 51 and the positioning member 52 are illustrated in FIGS. 16 and 17. In the electrode carrier/positioning member assembly 50' according to this embodiment of the present invention, the fitting SB is a tubular structure which is constituted by a strip of titanium or like biocompatible sheet metal arcuately bent into the form of a cage-like body defining two axially extending substantially parallel passageways 58a and 58b separated from each other by an interior transverse partition 58c. As in the case of the fittings 57 and 57', the passageway 58a accommodates the electrode carrier 51 and the passageway 58b accommodates the positioning member 52. The portion of the body of the fitting 58 through which the electrode carrier extends here is shown as being fixed to the latter by having a section 58d of the cage-forming strip molded into the electrode carrier somewhat rearwardly of the array of electrodes 55 (see FIG. 17), although the affixation could just as well be effected by crimping, while the portion of the body of the fitting through which the positioning member extends is dimensioned to permit longitudinal sliding movement of the positioning member. For the purposes of achieving the desired one-way movement of the positioning member relative to the fitting 58, the latter is provided with a pawl-like element 58e which extends from the partition 58c in the forward direction, is inclined at a small angle, on the order of about 10-15. or so, relative to the axis of the fitting and in a direction toward the axis of the passageway 58b, and terminates in a forwardly directed moderately sharp transverse distal edge 58f. The positioning member can, therefore, slide relatively freely past the pawl-like element 58e in the forward direction but, because of the pawl-like wedging action of the element 58e as it bites into the positioning member (see FIG. 17), cannot move relative to the fitting in the reverse direction.

Yet another type of fitting 59 for effecting the connection 54" between the trailing end regions 51a and 52a of the electrode carrier 51 and the positioning member 52 is illustrated in FIGS. 18 and 19. In the assembly 50" according to this embodiment of the present invention, the fitting 59 is constituted by a length of wire of titanium or a like biocompatible metal spirally wound into the form of cagelike body defining two axially extending substantially parallel passageways 59a and 59b separated from each other by an interior transverse partition 59c.

The passageway 59a accommodates the electrode carrier 51, and the passageway 59b accommodates the positioning member 52. In the illustrated assembly 50", the portion of the body of the fitting 59 through which the electrode carrier 51 extends is shown as being fixed to the latter by, e.g., having respective portions 59d of the cage-forming wire molded into the electrode carrier somewhat rearwardly of the trailing end of the array of electrodes 55, while that portion of the body of the fitting through which the positioning member 52 extends accommodates the positioning member for longitudinal sliding movement through the passageway 59b in both the forward and the reverse directions. For the purposes of ultimately locking the positioning member in this assembly against movement relative to the electrode carrier and the fitting in the reverse direction, the fitting must be sufficiently deformable so as to enable it to be crimped onto the positioning member (the locking action will, of course, be inherently effective against movement of the positioning member relative to the electrode carrier in either direction).

The electrode carrier/positioning member assembly 50. according to the embodiment of the present invention shown in FIG. 19 also includes, for effecting the desired linkage or articulated connection between the leading end regions 51a and 52a of the electrode carrier 51 and the positioning member 52, a ball-and-socket joint 53' which is a slightly modified version of the ball-and-socket joint 53 shown in FIG. 12. In the joint 53' the means for attaching the ball element 53a' to the electrode carrier comprises, in lieu of a bundle of wires, a flexible web or strand (or a bundle of strands) 56a' of Dacron polyester or a comparable biocompatible fibrous material, the web or strand being molded at one end thereof into the leading end region 51a of the electrode carrier and at its other end into the ball element. It should be understood, however, that of the two ball-and-socket joints herein disclosed, the joint 53 is preferred and currently constitutes what is deemed to be the best mode of effecting the linkage between the leading end regions of the electrode carrier and the positioning member in the assemblies 50, 50' and 50".

Referring now to FIG. 20, in the initial phase of the implantation of the electrode carrier/positioning member assembly 50 (or 50' or 50") into the cochlea 32, the leading end region of the assembly in its straight state is inserted into the scala tympani 36 through the round window 38, with the side region of the electrode carrier 51 where the contact faces of the electrodes 55 are exposed being directed toward the radially inner wall 43 of the cochlea and with the positioning member 52 lying at and along the side region of the electrode carrier which is directed toward the radially outer wall 44 of the cochlea. This phase of the insertion movement, which is effected by the surgeon pushing the positioning member longitudinally ahead with the aid of a suitable tool (not shown) and results in the electrode carrier being effectively pulled along by the positioning member due to the presence of the linkage 53 (not shown in FIGS. 20 and 21), continues until the bend in the leading end region 52a of the positioning member 52 comes into contact with the radially outer wall 44 of the cochlea, at which time a portion of the medial electrode-bearing region 51c of the electrode carrier is in contact with a portion of the radially inner wall 43 of the cochlea.

As the pushing force then continues to be exerted on the positioning member 52 by the surgeon, the assembly enters the spirally curved section of the cochlea and begins to adopt the curvature of the cochlea, with the positioning member continuing to glide along the radially outer wall 44 of the cochlea as indicated in phantom outline in FIG. 20. At the same time, however, the frictional drag being exerted by the radially inner wall 43 on the electrode carrier tends to retard the movement of the electrode carrier somewhat relative to the movement of the positioning member. Thus, the continuing pushing force that is exerted by the surgeon on the positioning member causes the trailing end region of the latter to advance somewhat relative to the electrode carrier past the location of the fitting 57 (or 57', 58 or 59). As a result, by virtue of the connections 53 and 54 (or 53' and 54' or 54") between the leading and trailing end regions of the electrode carrier and, respectively, the leading and trailing end regions of the positioning member and by virtue of the separation between the medial regions of the electrode carrier and the positioning member, the leading end region of the positioning member begins curving away from the outer wall 44 of the cochlea, as also shown in phantom outline in FIG. 20. With the positioning member exerting an outwardly directed force on the radially outer wall of the cochlea, the leading end region of the electrode carrier is forced across the width of the scala tympani as the medial region of the positioning member between its leading end region and the part of its trailing end region then extending through the fitting 57 (or 57', 58 or 59) begins to assume an outwardly bowed or arched configuration relative to the medial region of the electrode carrier.

By the time the assembly has reached the end of the spiral section of the cochlea, therefore, as is indicated diagrammatically in FIG. 21, the forces exerted by the positioning member through its leading and trailing end regions on the corresponding end regions of the electrode carrier ensure that not only the leading and trailing end regions of the electrode carrier but also its medial region over substantially the entire length of its electrode-bearing surface are shifted across the scala tympani into close hugging contact with the radially inner wall 43 of the cochlea, thereby disposing the electrodes 55 in as close a juxtaposition to the modiolus and the ganglion cells 40 as possible (see also FIG. 22) and not in the vicinity of the damaged hair cells (which is the situation illustrated in FIGS. 10 and 11). The positioning member thus not only achieves the desired disposition of the electrodes in proximity to the ganglion cells but also serves as the means for holding them in that disposition by virtue of the fact that the outward force exerted by the positioning member on the radially outer wall 44 of the cochlea ensures that the electrode carrier is continuously forced and held against the radially inner wall 43 of the cochlea.

Once the electrode carrier/positioning member assembly has been fully advanced into the cochlea to the desired final position of the electrode carrier, the fitting 57 (or 57' or 58), which is then located just outside the entrance to the cochlea, becomes a means for locking the entire assembly in place within the cochlea. This is achieved automatically and without the surgeon having to perform any positive act toward that end. As previously mentioned, the fitting 57 (or 57' or 58) effectively provides for a one-directional forward movement of the positioning member therethrough. Thus, in this case the positioning member can move substantially freely past the edge 57h (or 57h' or 58f) of the wedging element 57f (or 57f' or 58e) in the forward direction during the insertion stage, i.e., while the pushing force is being exerted on the positioning member by the surgeon. When the insertion stage has been completed, however, and the pushing force is relaxed, any reverse movement of the then bowed or arched positioning member is effectively precluded because as soon as such a reverse movement starts, the wedging element immediately bites into the positioning member (in the case of the fittings 57 and 57' assisted by the likewise functioning edges 57i and 57i'), thereby inhibiting any reverse movement thereof. Thus, since the medial region of the positioning member cannot straighten out and must remain in full surface contact over the major portion of its length with the radially outer wall of the cochlea, any instability or inadvertent movement of the assembly and especially of the electrode carrier in the cochlea is rendered effectively impossible.

In the case of the assembly 50", on the other hand, the same result is achieved except that the surgeon must perform a positive act in order to effect the locking action when the insertion of the assembly has been completed.

Thus, the surgeon must crimp the portion of the body of the fitting 59 through which the positioning member extends against the latter so as to prevent any further movement of the positioning member relative to the fitting and the electrode carrier in either direction. In this way, the assembly is constrained to remain firmly in its desired implanted position, because the medial region of the positioning member, being unable to straighten out, must remain in its arched state and hence in full surface contact over the major portion of its length with the radially outer wall of the cochlea. As a result, any instability or inadvertent movement of the electrode carrier in the cochlea, and especially a reverse movement thereof out of the cochlea, is rendered effectively impossible.

### Second Embodiment

Referring now to FIGS. 23-25, in the embodiment of the invention there shown the electrode carrier/positioning member assembly 60 includes an electrode carrier 61 and a positioning member 62 which are arranged alongside one another and include means 63 connecting the leading end region of the electrode carrier to the leading end region of the positioning member and means 64 connecting the trailing end region of the positioning member to the trailing end region of the electrode carrier somewhat rearwardly of the trailing end of the array of electrodes 65 on the electrode carrier. Whereas in the embodiment of FIGS. 20-22, however, both the electrode carrier and the positioning member are solid rods of the appropriate biocompatible plastic material, as shown in FIGS. 12-13, 17 and 19, in the embodiment of FIGS. 23-25 only the electrode carrier 61 is such a solid rod. The positioning member 62, on the other hand, is not a solid rod but rather is constituted by an elastic axially compressed tube 66 of a biocompatible plastic material, e.g., Silastic or the like, and a wire or tension member 67 of platinum or other biocompatible metallic or high strength plastic or fibrous material extending longitudinally slidably through the tube 66 for the purpose of imparting stiffness to the tube. The tube 66 even in its compressed state is somewhat longer than the array of electrodes 65.

As shown, the connection 63 between the leading end region 62a of the positioning member 62 and the leading end region 61 a of the electrode carrier 61 is effected by the leading end region of the wire 67 which protrudes from the leading end of the tube 66. The wire has a reverse bend 67a formed therein to define a hook-like portion the free end of which is secured, as shown at 67b, to the tip of the electrode carrier, e.g., by being molded thereinto. The bend 67a in the wire 67 at the same time constitutes a hinge portion about which the positioning member 62, under conditions to be described presently, can move angularly (pivotally) relative to the electrode carrier in the common axial plane of the assembly.

The connection 64 between the trailing end region 62b of the positioning member and the trailing end region 61b of the electrode carrier is effected by having the trailing end region 66b of the tube 66 fixedly secured, e.g.,, bonded, to the electrode carrier as shown at 68. The trailing end region of the wire 67 extends somewhat beyond the trailing end of the compressed tube and is initially held fast to the electrode carrier, e.g., by being clamped thereto (not shown) adjacent the location of the connection 64. In the starting condition of the assembly 60, therefore, the wire 67 maintains the compressed tube straight and in close parallel juxtaposition to the side region of the electrode carrier opposite to the side region where the electrodes 65 are exposed and thereby prevents the tube from expanding.

The initial insertion of the assembly 60 into the cochlea 32 (see FIG. 23) is effected, as before, through the round window 38 with both the electrode carrier 61 and the positioning member 62 in their straight state. During this phase of the insertion operation, for which the pushing force is applied by the surgeon to the electrode carrier, the trailing end of the wire 67 remains clamped to the electrode carrier, so that the wire is constrained against movement relative to the electrode carrier and ensures that the compressed tube also remains straight. When the assembly then enters the spirally curved section of the cochlea (see FIG. 24), both the positioning member 62 and the electrode carrier 61 become correspondingly curved as the positioning member rides along the radially outer wall 44 of the cochlea, during which time the electrode carrier (because of its previously described "memory" and tendency to return to its straight form and hug the outer wall of the cochlea) closely follows the curvature of the positioning member. The contact faces of the electrodes 65 are, consequently, disposed out of the desired close proximity to the radially inner wall 43 of the cochlea and the adjacent ganglion cells 40.

When the insertion operation has been carried out to the fullest extent possible, the assembly 60 over its entire length will be positioned as is shown in FIG. 24 for the leading end region thereof, i.e., with the electrodes 65 still positioned relatively far away from the ganglion cells 40. At that point, the trailing end of the wire is unclamped from the electrode carrier and released.

This eliminates the constraint exerted by the wire on the compressed tube 66 and permits the latter to expand longitudinally, but because both the leading and trailing end regions of the tube are stationarily connected to the electrode carrier at 63 and 64, the expansion of the tube causes it to assume an outwardly arched or bowed configuration relative to the electrode carrier with which the wire, by virtue of the hinge action of the bend 67a thereof and by virtue of the fact that a portion of the trailing end region of the wire is drawn into the tube as it expands and becomes longer, does not interfere. As a result, the expanding tube acts in the manner of a spring and, aided by the stiffening effect of the wire confined within the tube, exerts an outward force on the radially outer wall of the cochlea which causes the electrode carrier along its entire length to be forced across the width of the scala tympani, as indicated by the arrows in FIG. 25, until its concavely curved side region where the contact faces of the electrodes are located comes into contact with the radially inner wall of the cochlea and into close hugging contact with the modiolus, as shown in FIG. 25. The bowed or arched positioning member 62 not only serves to dispose the electrodes in the closest possible proximity to the ganglion cells, but also, once the trailing end region of the wire outside the tube has been re-clamped to the electrode carrier, locks the assembly, and especially the electrode carrier, in place and makes any instability or inadvertent movement thereof in the cochlea effectively impossible.

### Third Embodiment

Referring now to FIGS. 26-28, in this embodiment of the invention the electrode carrier/positioning member assembly 70 is a composite straight rodlike structure one part of which constitutes the electrode carrier 71 and another part of which constitutes the positioning member 72, the electrode carrier and the positioning member having their respective leading end regions 71a and 72a and their respective trailing end regions 71b and 72b connected to each other, e.g., by bonding or molding, at 73 and 74. The arrangement preferably is such that, with the positioning member 72 being located along the side region of the electrode carrier 71 opposite to the side region of the latter where the contact faces of the electrodes 75 are exposed, a small space 76 is left between and separates the medial regions of the electrode carrier and the positioning member from each other (although the presence of such a space is not essential as long as the two medial regions are not connected to each other). In the assembly 70, the electrode carrier 71 is as usual made of a biocompatible plastic material such as Silastic silicone polymer which does not have the property of swelling or expanding when exposed to water in the patient's body fluids. The positioning member 72, however, is made of a biocompatible plastic material which may also be Silastic silicone polymer but is compounded, e.g., by being admixed with finely ground NaCI, polyacrylic acid, or a like substance, so as to have the property of swelling or expanding when exposed to the water in the patient' s body fluids.

The assembly 70 is initially inserted into the scala tympani through the round window 38 while in its straight state (see FIG. 26). When the leading end region 72a of the positioning member 72 reaches the radially outer wall 44 of the cochlea and the assembly enters into the spirally curved section of the cochlea, the continuing pushing force exerted on the assembly by the surgeon causes the assembly to assume the curvature of the radially outer wall 44 of the cochlea. This can be seen from figure 27. In this embodiment, in contrast to the earlier embodiments, both the positioning member 72 and the electrode carrier 71 assume essentially the same curvature, even at full implantation, as they are joined as a single assembly 70. At full implantation, the components are still joined at both ends, and the electrode carrier 71 is positioned some distance from the radially inner wall 40.

However, as the positioning member 72 is formed from a water swellable material, due to exposure to the fluids in the scala tympani, the electrode carrier will expand and hence lengthen, as is shown in FIG 28. Positioning member 72 will accordingly urge, as shown by the arrows, electrode carrier 71 towards the radially inner wall 43. At portion 72a, the assembly remains joined, as it does at 74, so the expanding positioning member 72 forms curve 72b,separating from the electrode carrier 71. It can be seen that the resulting structure provides a similar function to the similar arching mechanical structures in the other embodiments. The material selected and geometry of the assembly 70 will determine the time taken to assume the expanded position. It will be appreciated that various altemative shapes could be used for the elements of the assembly 70 within this embodiment of the invention.

### Fourth Embodiment

FIGS. 29-31 show a cochlear electrode implant assembly 80 which, in accordance with the present invention, includes an electrode carrier 81 and an associated positioning member 82, together with means 83 for connecting the leading end region 81a of the electrode carrier to the leading end region 82a of the positioning member and with means 84 for connecting the trailing end region 81b of the electrode carrier to the trailing end region 82b of the positioning member while leaving the respective medial regions 81c and 82c of the electrode carrier and the positioning member separate from, i.e., unconnected to, each other. The electrode carrier 81 is shown as being a straight flexible solid rod of part circular or semicylindrical cross-section having a flat upper surface 81d and an arcuate, preferably circularly curved, lower surface 81 e, the rod being made of Silastic or an equivalent biocompatible material. The electrode carrier, in its medial region Sic between the leading and trailing end regions 81a and 81b, bears on its lower surface Ble an array of longitudinally spaced electrode elements 85 in the form of arcuate bands or layers of a biocompatible metal such as platinum or platinum alloy, and is further provided with a series of insulated electrical conductors 86 made of like biocompatible metals and embedded in the interior of the electrode carrier for conducting stimuli in the form of electrical sound-representing signals or impulses to the various electrode elements.

Correspondingly, the positioning member 82 in this embodiment of the invention is also a flexible solid rod of preferably part-circular or semicylindrical cross-section having a flat lower surface 82d and an arcuate, preferably circularly curved, upper surface 82e, this rod being shown as being somewhat thinner than the electrode carrier 81 (although this is not an essential or indispensable condition). The positioning member 82 initially is also generally straight, has its flat lower surface 82d juxtaposed to the flat upper surface 81d of the electrode carrier, i.e., to the side region of the latter opposite to the one where the contact faces of the electrode elements 85 are exposed, and extends lengthwise of and along the electrode carrier so as to have its leading and trailing end regions 82a and 82b adjacent, respectively, to the leading and trailing end regions 81a and 81b of the electrode carrier. The positioning member, for purposes of enhanced stiffness and tensile strength, is preferably made of a biocompatible material which is somewhat harder than Silastic, for example, nylon, Teflon™ (polytetrafluoroethylene) or a Teflon™-like material, or the like.

It will be understood, of course, that the electrode carrier and the positioning member may have otherwise matching configurations, for example, the former may be of cylindrical cross-section and the latter of a crescent or moonshaped cross-section, while the contacts or electrode elements may just as well have any of the configurations shown above.

The connection 83 between the leading end region 82a of the positioning member and the leading end region 81a of the electrode carrier in the illustrated embodiment of the present invention is effected, as best shown in FIGS. 29 and 33, by means of a latchless rod-and-socket joint which includes a socket part supported by the electrode carrier and a rod part supported by the positioning member.

More particularly, the electrode carrier 81 at the time of its manufacture is provided in its interior with a few, preferably from 2 to 4, additional insulated wires 87 (although one somewhat thicker wire could serve the purpose just as well) which are physically identical to and extend longitudinally through the electrode carrier like the conductors 86 but are not connected to the source of the electrical stimuli. The bundled wires 87 at their distal ends are arranged in a tubular coil formation 87a which is embedded in the tip region of the electrode carrier so as to have its axis or hollow interior slanting downwardly and forwardly from the flat upper surface 81d of the electrode carrier toward but not all the way to the arcuate lower surface 81 e thereof. Access to the interior of the wire coil formation is provided through an opening or port 81f which is formed in the upper surface 81 d of the electrode carrier and has its axis aligned with the axis of the coil formation 87a. Correspondingly, the positioning member 82 is provided at the time of its manufacture with an interior guide wire 88 (see FIG. 32) which extends along the entire length of the positioning member (see FIG. 29) and has its front end portion 88a projecting from the tip of the positioning member (see also FIG. 33). The front end portion 88a of the guide wire 88 is conventionally cross-sectionally round, having a diameter of about 0.1 mm, and projects for a distance of about 2 mm from the tip of the positioning member at an inclination to the longitudinal axis of the positioning member essentially matching the inclination of the axis of the coil formation 87a to the longitudinal axis of the electrode carrier. The main portion 88b of the guide wire 88, on the other hand, is flattened to a generally rectangular cross-section having a thickness of about 0.03 mm, and is disposed with its flat lower face 88c parallel to the flat lower surface 82d of the positioning member (see FIGS. 30 and 31).

The portion 88b of the guide wire 88, with a width about half that of the positioning member, thus serves to stiffen the latter against flexure in the plane of the guide wire while permitting essentially unhindered flexure in a direction perpendicular to the plane of the guide wire, i.e., toward and away from the flat upper surface 81d of the electrode carrier.

It will be understood, therefore, that the wire coil formation 87a in the electrode carrier constitutes the socket-part of the joint or connection 83, while the end portion 88a of the guide wire 88 projecting from the positioning member constitutes the rod-part of the joint. Moreover, in order to establish the joint during the assembly of the positioning member and the electrode carrier with each other, it is merely necessary to slip the projecting rod-shaped end portion 88a of the guide wire 88 into the coil formation 87a through the opening 81f in the upper surface 81d of the electrode carrier. It will also be apparent from the foregoing description that the joint 83 is devoid of any device for latching or locking the rod-part 88a to the socket-part 87a. Thus, a positive connection between the electrode carrier and the positioning member exists only when the latter is moving longitudinally forwardly while the rod-part 88a of the joint is freely slidingly received within the hollow interior of the socket-part 87a of the joint, in other words, during the insertion of the assembly into the cochlea.

When the positioning member is moved longitudinally rearwardly relative to the electrode carrier, the joint is automatically disabled as the rod-part 88a thereof, not being latched to the socket-part 87a, simply slips out of the socket-part and leaves the electrode carrier in place.

The connection 84 between the trailing end region 81b of the electrode carrier and the trailing end region 82b of the positioning member is effected by means of a fitting in the form of a ring-shaped band or like member 89 having an essentially D-shaped cross-sectional configuration characterized by a flat or planar inner wall 89a and an arcuate outer wall 89b, with the flat wall 89a of the 'D" defining the inner circumferential boundary surface of the ring. As can be seen from FIGS. 29 and 30, the inner configuration (form and diameter) of the ring member 89 closely matches the combined outer cross-sectional configuration of the assembly constituted by the electrode carrier 81 and the positioning member 82 regardless of their individual cross-sectional configurations so that, in their properly assembled state, the ring closely surrounds and embraces the trailing end regions of the positioning member and the electrode carrier and, despite the anti-friction properties of the material (PTFE or the like) of which the positioning member is made, frictionally holds the electrode carrier and the positioning member together. That frictional engagement, therefore, especially when the portion of the trailing end region 82b of the positioning member located just forwardly of the connection 84 is (see FIG. 29) disposed at an obliquely upward inclination to the electrode carrier and in the absence of any externally longitudinally applied force acting on the positioning member. suffices to restrain the positioning member against any free longitudinal displacement thereof through the ring and relative to the electrode carrier. The anti-friction properties of the material of which the positioning member is made at the same time ensure that when a longitudinal rearwardly directed force of an appropriate magnitude is exerted on the positioning member, the oblique portion of the latter located forwardly of the ring will be able to straighten out and slide through the ring relative to the electrode carrier.

Referring now to FIGS. 34-36, in the initial phase of the implantation of the electrode carrier/positioning member assembly 80 into the cochlea 32, the leading end region of the assembly in its straight state is inserted into the scala tympani 36 of the cochlea in the usual way (see FIG. 34), with the side region, i.e., the lower surface, of the electrode carrier 81 where the contact faces of the electrodes 85 are exposed being directed toward the radially inner wall 43 of the cochlea and with the positioning member 82 lying at and along the side region, i.e., the upper surface, of the electrode carrier which is directed toward the radially outer wall 44 of the cochlea. This phase of the insertion movement, which is effected by the surgeon pushing the positioning member longitudinally ahead, in the direction of the arrow A, with the aid of a suitable tool (not shown), results in the electrode carrier being effectively pulled along by the positioning member due to the presence of the joint 83. The forward movement of the assembly as a unit continues until the leading end region of the assembly comes into contact with the radially outer wall 44 of the cochlea, at which time a portion of the medial electrode-bearing region 81 c of the electrode carrier is in contact with a portion of the radially inner wall 43 of the cochlea.

As the pushing force then continues to be exerted on the positioning member 82 by the surgeon, the assembly enters the spirally curved section of the cochlea and begins to adopt the curvature of the cochlea, with the upper surface of the positioning member gliding along the radially outer wall 44 of the cochlea as indicated in FIG. 35. At the same time, however, the frictional drag being exerted by the radially inner wall 43 of the cochlea on the electrode carrier tends to retard the movement of the electrode carrier somewhat relative to the movement of the positioning member. Thus, the continuing pushing force, still in the direction of the arrow A, that is exerted by the surgeon on the positioning member causes the trailing end region of the latter to advance somewhat relative to the electrode carrier past the location of the ring or band 89. As a result, by virtue of the connections 83 and 84 between the leading and trailing end regions of the electrode carrier and, respectively, the leading and trailing end regions of the positioning member and by virtue of the separation between the medial regions of the electrode carrier and the positioning member, the leading end region 82a of the positioning member begins curving away from the outer wall 44 of the cochlea. With the positioning member exerting an outwardly directed force on the radially outer wall of the cochlea, the leading end region of the electrode carrier is forced across the width of the scala tympani as the medial region of the positioning member between its leading end region and the part of its trailing end region then extending through the ring member 89 begins to assume an outwardly bowed or arched configuration relative to the radial region of the electrode carrier.

By the time the assembly has reached the end of the spiral section of the cochlea, therefore, as is indicated diagrammatically in FIG. 36, the forces exerted by the positioning member through its leading and trailing end regions on the corresponding end regions of the electrode carrier ensure that not only the leading and trailing end regions of the electrode carrier but also its medial region over substantially the entire length of its electrode-bearing lower surface are shifted across the scala tympani into close hugging contact with the radially inner wall 43 of the cochlea, thereby disposing the electrodes 85 in as close a juxtaposition to the modiolus and the ganglion cells 40 as possible. The positioning member thus not only achieves the desired disposition of the electrodes in proximity to the ganglion cells but also serves as the means for holding them in that disposition by virtue of the fact that the outward force exerted by the positioning member on the radially outer wall 44 of the cochlea ensures that the electrode carrier is continuously forced and held against the radially inner wall 43 of the cochlea.

Once the electrode carrier/positioning member assembly has been fully advanced into the cochlea to the desired final position of the electrode carrier, the ring member 89, which is then located just outside the entrance to the cochlea, becomes a means for locking the entire assembly in place within the cochlea. This is achieved automatically and without the surgeon having to perform any positive act toward that end. At that time, as shown in FIG. 36, the upward or outward inclination of the portion of the trailing end region 82b of the positioning member 82 located just forwardly of the ring member 89 ensures that the latter will exert a frictional restraining action on the positioning member, thereby inhibiting any reverse movement thereof. Thus, since the medial region of the positioning member cannot straighten out and must remain in substantially full surface contact over the major portion of its length with the radially outer wall of the cochlea, any instability or inadvertent movement of the assembly and especially of the electrode carrier in the cochlea is rendered effectively impossible.

Referring now to FIGS. 37 and 38, the present invention greatly facilitates the removal of the implanted electrode assembly from the patient's ear if that becomes necessary for some reason. In that case, the surgeon needs only to hold the ring 89 stationary in the patient's outer ear and at the same time to exert on the positioning member 82 a rearwardly directed longitudinal pulling force, in the direction of the arrow B, of sufficient magnitude to overcome the frictional interaction between the ring and the obliquely inclined portion of the trailing end region of the positioning member. The resultant rearward sliding movement of that portion of the positioning member through the ring first straightens the arched portion of the positioning member down against the electrode carrier and ultimately causes the rod-part 88a of the joint 83 to be pulled freely out of the associated coil-shaped socket-part 87a of the joint. Thereafter, the then disconnected leading end region 82a and the medial region 82c of the positioning member move rearwardly along the radially outer wall 44 of the cochlea while in sliding contact with that wall, as shown in FIG. 37, which permits the positioning member to be easily withdrawn from the cochlea.

The immediate effect of this extraction of the positioning member on the electrode carrier 81 will then depend on whether or not scar tissue has grown in the cochlea and encapsulated the electrode carrier. If such encapsulation (not shown) has taken place, the electrode carrier will remain in its operational adjusted position, as shown in solid lines in FIG. 38, with the electrodes or contact elements 85 still in close proximity to the modiolus 43 and the ganglion cells 40. On the other hand, if no encapsulation has taken place, then after the connection 83 has been broken and the positioning member has been withdrawn, the 'memory of the material of which the electrode carrier is made causes the latter to try to uncoil and straighten out, with the result that the upper surface of the electrode carrier comes into contact with the radially outer wall of the cochlea, as shown in broken lines in FIG. 38. In either condition, of course, the electrode carrier can be easily extracted from the cochlea.

It will be understood that the foregoing description of a number of embodiments of the present invention is for purposes of illustration only, and that the various structural and operational features and relationships herein disclosed are susceptible to a number of modifications and changes within the scope of the claims appended hereto.

## Claims

1. A self-positioning cochlear electrode implant assembly adapted to be implanted in the scala tympani of the spirally curved cochlea of a human ear for stimulating cells of the spiral ganglion, said assembly comprising:
a rod-shaped cochlear electrode carrier (51) made of a biocompatible plastic material and sufficiently flexible to be able to assume the spiral curvature of the cochlea, said electrode carrier (51) having leading (51a) and trailing (51b) end regions and tapering in cross-section from said trailing end region (51b) to said leading end region (51a); an array of cochlear electrode elements (55) supported by said electrode carrier (51) at respective longitudinally spaced locations thereon in a medial region of said electrode carrier (51) between said leading and trailing end regions thereof, said electrode elements having respective contact faces exposed along a first longitudinal side region of said electrode carrier (51); and a rod-shaped positioning member (52); **characterised in that** said positioning member (52) is made of a biocompatible plastic material and sufficiently flexible to be able to assume the spiral curvature of the cochlea, said positioning member having leading (52a) and trailing (52b) end regions and being juxtaposed to and extending lengthwise of said electrode carrier (51) along a second longitudinal side region of the latter opposite said first longitudinal side region thereof so as to have said leading (52a) and trailing (52b) end regions of said positioning member (52) located adjacent said leading (51a) and trailing (51b) end regions, respectively, of said electrode carrier (51); and means (53,54) connecting said leading (52a) and trailing (52b) end regions of said positioning member (52) to said leading (51a) and trailing (51b) end regions, respectively, of said electrode carrier (51) so as to leave a medial region (52c) of said positioning member separated from said medial region (51c) of said electrode carrier (51) and adapted to assume an arched configuration relative to said medial region (51c) of said electrode carrier (51);
whereby, upon insertion of said assembly into the scala tympani with said positioning member (52) engaging and closely following the curvature of the radially outer wall of the cochlea and with said electrode carrier (51) juxtaposed to and closely following the curvature of said positioning member (52), said positioning member (52) when assuming said arched configuration against the constraint of the radially outer wall of the cochlea exerts a force on said electrode carrier (51) at said leading (51a) and trailing (51b) end regions thereof to shift said electrode carrier (51) into engagement of said first longitudinal side region thereof with the radially inner wall of the cochlea so as to position said contact faces of said array of electrode elements (55) in close proximity to the spiral ganglion cells of the cochlea; and the connection between said trailing end region of said electrode carrier and said trailing (51b) end region of said positioning member (52) serves, when the insertion of said assembly into the cochlea has been completed and said positioning member (52) is in said arched configuration thereof, to stabilize said assembly in and lock the same against inadvertent reverse movement out of the cochlea.

2. A cochlear electrode implant assembly according to claim 1, wherein said means (54) for connecting said trailing end regions (51b,52b) of said electrode carrier (51) and said positioning member (52) to each other comprises a fitting having a body (57) with at least partly arcuate exterior peripheral portions (57a,57b), forward and rearward ends, and an interior partition (57e) extending from one end of said body to the other to provide said fitting with first and second substantially parallel passageways (57c,57d) extending therethrough for accommodating said electrode carrier (51) and said positioning member (52), respectively; the portion of said body of said fitting which in conjunction with said partition (57e) defines one of said first and second passageways (57c,57d) is permanently affixed to the respective one of said electrode carrier (51) and said positioning member (52) extending through said one passageway; the portion of said body (57) of said fitting which in conjunction with said partition (57e) defines the other of said first and second passageways is dimensioned for permitting sliding relative movement between said fitting and the respective other of said electrode carrier (51) and said positioning member (52) extending through said other passageway; and said portion of said body (57) of said fitting in which said other passageway is located is provided with means enabling said other of said electrode carrier (51) and said positioning member (52) to be locked against movement through said other passageway.

3. A cochlear electrode implant assembly according to claim 2, wherein said second passageway (57d) is dimensioned to permit sliding relative movement between said fitting and said positioning member (52) and the portion of said body (57) of said fitting in which said second passageway (57d) is located is provided with said means enabling said positioning member (52) to be locked against movement through said second passageway (57b).

4. A cochlear electrode implant assembly according to claim 2 or 3, wherein the portion of said body (57) of said fitting in which said first passageway (57c) is located is permanently fixed to said electrode carrier (51).

5. A cochlear electrode implant assembly according to claim 2 or 3, wherein said portion of said body (57) of said fitting in which said second passageway (57d) is located is provided with wedge-like means permitting forward movement but preventing reverse movement of said positioning member (52) through said second passageway (57d).

6. A cochlear electrode implant assembly according to claim 5, wherein said wedge-like means (57f) comprises a pawl-like member (57f) supported by and extending forwardly from said portion of said body (57) of said fitting in which said second passageway (57d) is located, said pawl-like member (57f) being inclined toward the axis of said second passageway (57d) and having a forward end edge (57h) which is in sliding engagement with a first surface region of said positioning member (52) when the latter moves forwardly through said second passageway, said forward end edge of said pawl-like member (57f) being sufficiently sharp to bite into said first surface region of said positioning member (52) and thereby wedge said positioning member (52) against reverse movement through said second passageway (57d) substantially immediately upon start of such reverse movement.

7. A cochlear electrode implant assembly according to claim 6, wherein said pawl-like member (57f) is supported by and extends forwardly from said partition (57e) at an inclination toward the axis of said second passageway (57d)

8. A cochlear electrode implant assembly according to claim 7, wherein pawl-like member (57f) is supported by said partition (57e) adjacent the rearwardmost end of said partition and extends forwardly therefrom within the confines of said second passageway (57d).

9. A cochlear electrode implant assembly according to claim 8, wherein said pawl-like member (57f) is welded to said partition (57e).

10. A cochlear electrode implant assembly according to claim 8, wherein said pawl-like member (57f) is an integral extension of said partition (57e) at said rearwardmost end of said partition and is bent over therefrom into said second passageway (57d).

11. A cochlear electrode implant assembly according to claim 8, wherein said portion of said body (57) of said fitting in which said second passageway (57d) is located has a forward end edge (57i) which is spaced from said forward end edge (57h) of said pawl-like member (57e) and in sliding engagement with a second surface region of said positioning member (52), and which is sufficiently sharp to bite into said second surface region of said positioning member (52) and thereby assist in wedging said positioning member (52) against reverse movement through said second passageway (57d) substantially immediately upon start of such reverse movement.

12. A cochlear electrode implant assembly according to claim 1, wherein each of said electrode carrier (51) and said positioning member (52) has the form of a solid rod; and said means (53) connecting said leading end regions of said electrode carrier (51) and said positioning member (52) to each other comprises a ball-and-socket joint (53) including a ball component (53a) and a correspondingly configured socket component (53c) each provided on a respective one of said electrode carrier (51) and said positioning member (52).

13. A cochlear electrode implant assembly according to claim 12, wherein said ball component (53a) of said joint (53) is located on said electrode carrier (51), and said socket component (53c) of said joint (53) is located on said positioning member (52).

14. A cochlear electrode implant assembly according to claim 13, wherein said ball component (53a) of said joint (53) comprises a plurality of bundled wires of a biocompatible metallic material which at a first end region thereof are embedded in said electrode carrier (51) and which project from said leading end (51a) region of said electrode carrier (51) generally longitudinally thereof, and said bundled wires exteriorly of said electrode carrier (51) terminate in a second end region and thereat support a ball member; said socket component of said joint comprises an axial rearwardly extending slit provided in said leading end region (52b) of said positioning member (52) in a common axial plane of both said positioning member (52) and said electrode carrier (51), and a ball member-receiving surface recess or depression provided on said positioning member (52) on a side region of the latter which is directed away from said electrode carrier (51) and at a location straddling said slit, said slit being dimensioned to accommodate the portion of said bundled wires between said first and second end regions of the latter; and said joint is established by said portion of said bundled wires being received in said slit and said ball member being received and seated in said recess or depression.

15. A cochlear electrode implant assembly according to claim 1, wherein each of said electrode carrier (51) and said positioning member (52) has the form of a solid rod; said means (54) connecting said trailing end regions (51b,52b) of said electrode carrier (51) and said positioning member (52) to each other comprises a tubular fitting (59) having a body provided with an internal generally axial partition (59c) and defining on opposite sides of said partition first and second substantially parallel passageways (59a,59b) extending through and generally axially of said tubular fitting (59) for accommodating said electrode carrier (51) and said positioning member (52), respectively; the portion of said body (57) of said tubular fitting (59) which defines said first passageway (59a) is permanently fixed to said electrode carrier (51) for joint movement of said tubular fitting (59) (59) with said electrode carrier (51); the portion of said body (57) of said tubular fitting (59) which defines said second passageway (59b) is dimensioned to permit sliding movement of said positioning member (52) relative thereto; and said tubular fitting (59) is provided with means adapted to be activated upon completion of the insertion of said assembly into the cochlea for immobilizing said positioning member (52) in said arched configuration thereof against reverse movement of said positioning member (52) relative to said tubular fitting (59) and for thereby stabilizing said assembly in the cochlear.

16. A cochlear electrode implant assembly according to claim 15, wherein the portion of said body (57) of said tubular fitting (59) which defines said second passageway (59b) is deformable to permit crimping thereof onto said positioning member (52), and said deformable portion of said body (57) of said tubular fitting (59) constitutes said means activatable for immobilizing said positioning member (52).

17. A cochlear electrode implant assembly according to claim 16, wherein said tubular fitting (59) comprises a length of wire spirally wound to define both an outer wall of said body (57) of said tubular fitting (59) and said internal partition (59c) thereof between said first and second passageways.

18. A cochlear electrode implant assembly according to claim 15, wherein the portion of said body (57) of said tubular fitting (59) which defines said internal partition (59c) thereof is provided with a wedge-like structure extending from said partition toward the axis of said second passageway for engagement with said positioning member (52) when the latter is accommodated in said second passageway, said wedge-like structure is arranged to permit forward movement and to block reverse movement of said positioning member (52) through said second passageway, and said wedge-like structure constitutes said means activatable for immobilizing said positioning member (52).

19. A cochlear electrode implant assembly according to claim 18, wherein said wedge-like structure comprises a pawl-like member projecting forwardly from said partition and inclined at an angle toward said axis of said second passageway, said pawl-like member having a forward edge which does not interfere with forward movement of said positioning member through said second passageway but which is able to bite into said positioning member for blocking any reverse movement of the latter.

20. A cochlear electrode implant assembly according to claim 19, wherein said tubular fitting comprises a strip of metal wound to define both an outer wall of said body of said tubular fitting and said internal partition thereof between said first and second passageways, and the portion of said strip which constitutes said partition has a lateral angularly inclined extension which constitutes said pawl-like member.

21. A cochlear electrode implant assembly according to claim 1, wherein said electrode carrier (51) has the form of a solid rod; said positioning member (52) comprises an elastic axially compressed tube the length of which between its leading and trailing end regions in its compressed state is somewhat greater than the length of said array of electrode elements (55) on said electrode carrier (51), and a flexible tension member extending slidably through said tube and having first and second end regions projecting from said tube at said leading and said trailing end regions, respectively, of said tube; said means for connecting said leading end regions (51a) of said electrode carrier (51) and said positioning member (52) to each other comprises a fixed connection between said first end region of said tension member and said leading end region of said electrode carrier (51); and said means connecting said trailing end regions of said electrode carrier (51) and said positioning member (52) to each other comprises a fixed connection between said trailing end region of said tube and said trailing end region of said electrode carrier (51), and means for releasably attaching said second end region of said tension member to said electrode carrier (51) outside and somewhat rearwardly of said fixed trailing end region of said tube; whereby said attaching means, when activated with said tension member straight prior to the insertion of said assembly into the cochlea, serves to maintain said positioning member (52) in close juxtaposition to said electrode carrier (51) while permitting both said electrode carrier (51) and said positioning member (52) jointly to assume the curvature of the cochlea; when deactivated after insertion of said assembly into the cochlea, serves to release said tension member so as to permit said tube, while stiffened by said tension member, to expand and assume said arched configuration and to shift said electrode carrier (51) within the cochlea so as to juxtapose said contact faces of said array of electrode elements to the spiral ganglion cells; and when reactivated with said positioning member (52) in said arched configuration thereof, serves to immobilize said tension member so as to maintain said positioning member (52) in said arched configuration for thereby to stabilizing said assembly in the cochlea.

22. A cochlear electrode implant assembly according to claim 21, wherein said tension member is a wire of a biocompatible metal.

23. A cochlear electrode implant assembly according to claim 1, wherein each of said electrode carrier (51) and said positioning member (52) has the form of a solid rod, said electrode carrier (51) being made of a biocompatible plastic material which is resistant to swelling or expanding when exposed to water, and said positioning member (52) being made of a biocompatible plastic material which has the property of swelling or expanding when exposed to water; and said means connecting said leading and trailing end regions of said electrode carrier (51) to said leading and trailing end regions, respectively, of said positioning member (52) comprise a pair of fixed connections therebetween; whereby during the insertion of said assembly into the cochlea, said electrode carrier (51) and said positioning member (52) remain in close juxtaposition to each other and jointly assume the curvature of the cochlea; after the insertion, upon said assembly being exposed over a period of time to the action of the water in the person's body fluid present in the cochlea, said positioning member (52) expands and assumes said arched configuration and shifts said electrode carrier (51) within the cochlea so as to juxtapose said contact faces of said array of electrode elements (55) to the spiral ganglion cells; and said positioning member (52) in said arched configuration serves to immobilize said assembly for stabilizing the same in the cochlea.

24. A cochlear electrode implant assembly according to claim 23, wherein said fixed connections between said electrode carrier (51) and said positioning member (52) are molded or bonded joints.

25. A cochlear electrode assembly according to claim 1, wherein said means (53) connecting said leading end regions of said electrode carrier (51) and said positioning member (52) to each other comprise a latchless rod-and-socket joint including a rod part supported by one of said positioning member (52) and electrode carrier (51) and a correspondingly configured socket-part supported by the other of said positioning member (52) and electrode carrier (51), said socket part being constructed for slidingly receiving said rod-part without the two parts becoming latched to each other.

26. A cochlear electrode assembly according to claim 25, wherein said means for connecting said trailing end regions of said electrode carrier (51) and said positioning member (52) to each other comprises a ring-shaped member made of a biocompatible plastic material and defining a passageway extending therethrough dimensioned for jointly accommodating said electrode carrier (51) and said positioning member (52) with a close fit; and said ring-shaped member has a cross-sectional configuration defining a circumferential boundary surface for said passageway which, when said positioning member (52) is in said arched state thereof and in the absence of a longitudinally and rearwardly directed force of an appropriate magnitude externally applied by the surgeon to said positioning member (52), frictionally restrains said positioning member (52) against inadvertent reverse movement through said ring-shaped member and out of the cochlea.

27. A cochlear electrode implant assembly according to claim 26, wherein said cross-sectional configuration of said ring-shaped member is generally D-shaped, with the flat wall of the D defining said circumferential boundary surface of said passageway.

28. A cochlear electrode implant assembly according to any one of claims 25 to 27, wherein said socket-part of said joint is supported by said electrode carrier (51), and said rod-part of said joint is supported by said positioning member (52).

29. A cochlear electrode implant assembly according to claim 28, wherein said socket-part of said joint comprises a wire coil formation made of a biocompatible metallic material and embedded in said leading end region of said electrode carrier (51), said wire coil formation has an axis and an axial hollow interior for receiving said rod-part of said joint, and said axis of said wire coil formation is obliquely inclined within said electrode carrier (51) rearwardly and upwardly in the direction from said first to said second longitudinal side region thereof; said electrode carrier (51) is provided at said second longitudinal side region thereof with an access opening aligned and communicating with said hollow interior of said wire coil formation; said rod-part of said joint comprises a rod-shaped element made of a biocompatible metallic material and projecting forwardly from said leading end region of said positioning member (52), and said rod-shaped element is obliquely inclined relative to said positioning member (52) forwardly and downwardly in the direction from said positioning member (52) toward said electrode carrier (51) when the same are located adjacent each other in said assembly; and said joint is established by said rod shaped element slidingly entering said hollow interior of said wire coil formation through said access opening, whereby only a forward movement of said positioning member (52) causes a pushing force to be exerted by said rod-shaped element on said wire coil formation so as to cause a forward movement of said electrode carrier (51), while a rearward movement of said positioning member (52) causes said rod-shaped element to be withdrawn from said wire coil formation and leaves said electrode carrier (51) stationary.

30. A cochlea electrode implant assembly according to claim 29, wherein said positioning member (52) has a guide wire (67)embedded therein and extending through the entire length of said positioning member (52), the body of said guide wire (67)within said positioning member (52) is substantially flat in cross sectional configuration and has a flat face thereof facing toward said second longitudinal side region of said electrode carrier (51) when said positioning member (52) and said electrode carrier (51) are located adjacent each other in said assembly, said guide wire (67)thereby stiffening said positioning member (52) against flexure in a direction in the plane of said guide wire (67)while permitting unhindered flexure of said positioning member (52) in a direction perpendicular to the plane of said guide wire (67), and said rod-shaped element is constituted by an end region of said guide wire (67)located outside said positioning member (52).

31. A cochlear electrode implant assembly according to claim 30, wherein the width of said body (57) of said guide wire (67) is about one-half the width of said positioning member (52) in a side region of the latter which is located proximate to said second longitudinal side region of said electrode carrier (51) when said positioning member (52) and said electrode carrier (51) are adjacent each other in said assembly.

32. A cochlear electrode implant assembly according to claim 30, wherein the portion of said guide wire (67) which constitutes said rod-shaped element is of conventionally round cross-sectional configuration.

33. A cochlear implant device adapted for insertion in the scala tympani, including an electrode carrier (51) including a plurality of electrodes (55), and having leading and trailing ends, (51a,b) wherein said device further comprises a positioning member (52), **characterised in that** said electrode carrier (51) is not pre-stressed before being inserted into the scala tympani, said positioning member (52) being adapted to be joined to said electrode carrier (51) at least during insertion at said leading end of said electrode carrier (51) so as to guide said electrode carrier (51) during insertion, and after insertion said positioning member (52) is adapted to assume an arched configuration relative to said electrode carrier (51), with a medial region of the positioning member between the leading and trailing ends being separated from the juxtaposed medial region of the electrode carrier (51), so as to urge said electrode carrier (51) towards the radially inner wall of said scala tympani, said device further including means (54) for connecting the trailing end (51b) of said electrode carrier (51) to said positioning member (52) so that said arched configuration is maintained.

34. A cochlear implant device according to claim 33, wherein said positioning member (52) is initially attached only at the leading end (52a) to said electrode carrier (51), and after insertion said means for connecting the trailing end are attached.

35. A cochlear implant device according to claim 34, wherein said positioning member (52) is attached at the leading end (51a) of said electrode carrier (51) by attachment means which detach on reverse movement of said positioning means, so as to allow the positioning means to be detached from the electrode carrier (51) to facilitate removal of the electrode carrier (51).

36. A cochlear implant device according to claim 35, wherein the attachment means (53) is a latchless rod and socket joint.

37. A cochlear implant device according to claim 33, wherein said electrode carrier (51) is initially attached at both ends to said positioning member (52), said positioning member (52) operatively increasing in length so as to form said arched configuration.

38. A cochlear implant device according to claim 37, wherein said increase in length is due to water swelling of the positioning member (52).

## Patentansprüche

1. Selbstpostitionierende Elektrodenimplantatanordnung für eine Cochlea, angepasst zur Implantierung in der Scala Tympani der spiralförmig gebogenen Cochlea eines menschlichen Ohres für die Anregung von Zellen des spiralförmigen Ganglions, wobei die Anordnung Folgendes aufweist:
einen stabförmig gestalteten cochlearen Elektrodenträger (51), hergestellt aus einem bioverträglichen Kunststoffmaterial und ausreichend biegbar, um die spiralförmige Krümmung der Cochlea anzunehmen, wobei der Elektrodenträger (51) Führungs-(51a) und Schlepp-Endbereiche aufweist und sich im Querschnitt verjüngt von dem nachlaufenden Endbereich (51b) bis zu dem Eührungsendbereich (51a);
eine Anordnung von cochlearen Elektrodenelementen (55), die durch den Elektrodenträger (51) unterstützt ist an entsprechenden in Längsrichtung beabstandeten Positionen desselben in einem mittleren Bereich des Elektrodenträgers (51) zwischen den Führungs- und nachfolgenden Endbereichen desselben, wobei die Elektrodenelemente jeweilige Kontaktflächen aufweisen, die offen positioniert entlang eines ersten Längsseitenbereiches des Elektrodenträgers (51) sind; und
ein stabförmig gestaltetes Positionierungsglied (52);
**dadurch gekennzeichnet, dass**
das Positionierungsglied (52) aus einem bioverträglichen Kunststoffmaterial besteht und ausreichend flexibel ist, um die spiralförmige Krümmung der Cochlea anzunehmen, wobei das Positionierungsglied Führungs-(52a) und Schlepp-(52b)Endbereiche aufweist und angrenzt an und sich längsseitig des Elektrodenträgers (51) erstreckt entlang eines zweiten Längsseitenbereiches des Letzteren gegenüberliegt dem ersten Längsseitenbereich desselben, damit die Führungs-(52a) und Schlepp-(52b)Endbereiche des Positionierungsgliedes (52) angrenzend angeordnet sind zu den Führungs-(51a) und Schlepp-(51b)Endbereichen des Elektrodenträgers (51) in entsprechender Weise; und Mitteln (53, 54) zur Verbindung der Führungs-(52a) und Schlepp-(52b)Endbereiche des Positionierungsgliedes (52) mit den Führungs-(51a) und Schlepp-(51b)Endbereichen des Elektrodenträgers (51) in entsprechender Weise, um einen mittleren Bereich (52c) des Positionierungsgliedes getrennt zu halten von dem mittleren Bereich (51c) des Elektrodenträgers (51) und derart angepasst, um eine gebogene Gestaltung relativ zu dem mittleren Bereich (51c) des Elektrodenträgers (51) anzunehmen;
wobei auf die Einführung der Anordnung in den Scala Tympani, mit dem Ineinandergreifen und der genauen Verfolgung der Krümmung der radialen äußeren Wand der Cochlea durch das Positionierglied (52) und mit der angrenzenden Positionierung und der genauen Verfolgung der Krümmung des Positioniergliedes (52) durch den Elektrodenträger (51) das Positionierglied (52), wenn die gebogene Gestaltung der radialen äußeren Wand der Cochlea zwangsläufig angenommen wird, eine Kraft auf den Elektrodenträger (51) an den Führungs-(51a) und Schlepp-(51b)Endbereichen desselben ausübt, um den Elektrodenträger (51) in den Eingriff mit dem ersten Längsseitenbereich desselben mit der radialen inneren Wand der Cochlea zu verschieben, um die Kontaktflächen der Anordnung der Elektrodenelemente (55) nahe bei den Zellen des Spiralganglions der Cochlea zu positionieren; und die Verbindung zwischen dem Führungsendbereich des Elektrodenträgers und dem Führungs-(51b)Endbereich des Positioniergliedes (52) dienen, wenn die Einführung der Anordnung in die Cochlea abgeschlossen ist und das Positionierglied (52) sich in der gebogenen Gestaltung desselben befindet zur Stabilisierung der Anordnung darin und zum Verriegeln derselben gegen versehentliche rückwärtige Bewegungen aus der Cochlea heraus.

2. Elektrodenimplantatanordnung für eine Cochlea nach Anspruch 1, worin das Mittel (54) zur Verbindung der Schlepp-Endbereiche (51b, 52b) des Elektrodenträgers (51) und des Positioniergliedes (53) gegeneinander eine Halterung mit einem Körper (57) aufweist mit zumindest teilweise gebogenen äußeren peripheren Teilen (57a, 57b), vorderen und rückwärtigen Enden, und einer inneren Zwischenwand (57e), die sich vom einen Ende des Körpers zum anderen erstreckt, um die Halterung mit den ersten und zweiten, im Wesentlichen parallelen Durchgängen (57c, 57d) zu versehen, die sich hindurch zur Aufnahme des Elektrodenträgers (51) und des Positioniergliedes (52) in entsprechender Weise erstrecken; worin der Teil des Körpers der Halterung der in Verbindung mit der Zwischenwand (57e) einen der ersten und zweiten Durchgänge (57c, 57d) bestimmt, ständig befestigt ist mit dem entsprechenden Ende des Elektrodenträgers (51) und dem Positionierglied (52), welches sich durch den einen Durchgang hindurch erstreckt; worin der Teil des Körpers (57) der Halterung, der in Verbindung mit der Zwischenwand (57e) den anderen der ersten und zweiten Durchgänge bestimmt, derart ausgelegt ist, um eine gleitende relative Bewegung zu erlauben zwischen der Halterung und dem entsprechend anderen Elektrodenträger (51) wobei das Positionierglied (52), sich durch den anderen Durchgang hindurch erstreckt; und worin das Teil des Körpers (57) der Halterung, in dem der andere Durchgang positioniert ist, mit Mitteln ausgestattet wird, die es ermöglichen, dass der andere der Elektrodenträger (51) und das Positionierglied (52) verriegelt sind gegen eine Bewegung durch einen anderen Durchgang.

3. Elektrodenimplantatanordnung für eine Cochlea entsprechend Anspruch 2, worin der zweite Durchgang (57d) derart ausgelegt ist, um eine gleitende relative Bewegung zu ermöglichen zwischeh der Halterung und dem Positionierglied (52) und das Teil des Körpers (57) der Halterung in dem der zweite Durchgang (57d) platziert ist, ist ausgestattet mit den Mitteln zur Ermöglichung der Verriegelung des Positioniergliedes (52) gegen eine Bewegung durch den zweiten Durchgang (57b).

4. Elektrodenimplantatanordnung für eine Cochlea nach Anspruch 2 oder 3, worin das Teil des Körpers (57) der Halterung, in welchem der erste Durchgang (57c) positioniert ist, ständig an dem Elektrodenträger (51) befestigt ist.

5. Elektrodenimplantatanordnung für eine Cochlea nach Anspruch 2 oder 3, worin das Teil des Körpers (57) der Halterung, in welcher der zweite Durchgang (57d) positioniert ist, ausgestattet ist mit keilförmigen Mitteln, die eine Vorwärtsbewegung erlauben, jedoch eine Rückwärtsbewegung des Positioniergliedes (52) durch den zweiten Durchgang (57d) verhindern.

6. Elektrodenimplantatanordnung für eine Cochlea nach Anspruch 5, worin das keilförmige Mittel (57f) ein Sperrglied (57f) aufweist, das unterstützt wird durch und sich nach vorne erstreckt von dem Teil des Körpers (57) der Halterung, in welchem der zweite Durchgang (57d) positioniert ist, wobei das Sperrmittel (57f) bezüglich der Achse des zweiten Durchganges (57d) geneigt ist und einen vorderen Rand (57h) aufweist, der sich in gleitendem Eingriff mit einem ersten Oberflächenbereich des Positioniergliedes (52) befindet, wenn letzteres sich nach vorne bewegt durch den zweiten Durchgang, wobei der vordere Rand des Sperrgliedes (57f) ausreichend scharf ist, um in den ersten Oberflächenbereich des Positioniergliedes (52) zu schneiden und dabei das Positionierglied (52) einzukeilen gegen rückwärtige Bewegung durch den zweiten Durchgang (57d) im Wesentlichen unmittelbar nach dem Start einer solchen Rückwärtsbewegung.

7. Elektrodenimplantatanordnung für eine Cochlea nach Anspruch 6, worin das Sperrglied (57f) unterstützt wird durch und sich nach vorne von der Zwischenwand (57e) erstreckt mit einer Neigung relativ zur Achse des zweiten Durchganges (57d).

8. Elektrodenimplantatanordnung für eine Cochlea nach Anspruch 7, worin das Sperrglied (57f) unterstützt wird durch die Zwischenwand (57e), welches benachbart ist zum äußersten rückwärtigen Ende der Zwischenwand und sich davon vorwärts erstreckt innerhalb der Grenzen des zweiten Durchganges (57d).

9. Elektrodenimplantatanordnung für eine Cochlea nach Anspruch 8, worin das Sperrglied (57f) mit der Zwischenwand (57e) verschweißt ist.

10. Elektrodenimplantatanordnung für eine Cochlea nach Anspruch 8, worin das Sperrglied (57f) eine integrale Verlängerung der Zwischenwand (57e) am äußerst rückwärtigen Ende der Zwischenwand ist und davon umgebogen ist in den zweiten Durchgang (57d).

11. Elektrodenimplantatanordnung für eine Cochlea nach Anspruch 8, worin das Teil des Körpers (57) der Halterung, in dem der zweite Durchgang (57d) positioniert ist, einen vorderen Rand (57i) aufweist, der beabstandet ist von dem vorderen Rand (57h) des Sperrgliedes (57e) und sich in gleitendem Eingriff befindet mit einem zweiten Oberflächenbereich des Positioniergliedes (52), und welches ausreichend scharf ist, um in den zweiten Oberflächenbereich des Positioniergliedes (52) einzuschneiden und dadurch das Verkeilen des Positioniergliedes (52) gegen Rückwärtsbewegung durch den zweiten Durchgang (57d) im Wesentlichen unmittelbar nach Start der Rückwärtsbewegung zu unterstützen.

12. Elektrodenimplantatanordnung für eine Cochlea nach Anspruch 1, worin sowohl der Elektrodenträger (51) als auch das Positionierglied (52) die Form eines stabilen Stabes aufweisen; und das Mittel (53), welches die Führungsendbereiche des Elektrodenträgers (51) und des Positioniergliedes (52) miteinander verbindet, enthält ein Kugelgelenk (53) mit einem Kugelbestandteil (53a) und ein korrespondierend ausgebildetes Muffenbestandteil (53c), jedes ausgebildet auf dem entsprechenden Elektrodenträger (51) und dem Positionierelement (52).

13. Elektrodenimplantatanordnung für eine Cochlea nach Anspruch 12, worin der Kugelbestandteil (53a) der Verbindung (53) auf dem Elektrodenträger (51) positioniert ist und der Muffenbestandteil (53c) der Verbindung (53) auf dem Positionierglied (52) positioniert ist.

14. Elektrodenimplantatanordnung für eine Cochlea nach Anspruch 13, worin der Kugelbestandteil (53a) der Verbindung (53) eine Vielzahl von gebündelten Drähten aus einem bioverträglichen metallischen Material aufweist, welche an einem ersten Endbereich desselben in dem Elektrodenträger (51) eingebettet sind und welche allgemein in Längsrichtung aus dem Führungs-(51a)Endbereich des Elektrodenträgers (51) heraustreten, und worin die gebündelten Drähte außerhalb des Elektrodenträgers (51) enden in einem zweiten Endbereich und dort ein Kugelglied tragen; worin der Muffenbestandteil der Verbindung einen axialen, sich rückwärtig erstreckenden Schlitz aufweist, in dem Führungs-(52b)Endbereich des Positioniergliedes (52) in einer gemeinsamen axialen Ebene sowohl des Positioniergliedes (52) als auch des Elektrodenträgers (51), und eine Kugelgliedaufnahmeoberfläche mit Vertiefung oder Mulde, die bereitgestellt ist auf dem Positionierglied (52) auf einem Seitenbereich des letzteren, welche von dem Elektrodenträger (51) weg gerichtet ist und an einer Position an der der Schlitz spreizt wird, wobei der Schlitz zur Aufnahme des Teiles der gebündelten Drähte zwischen dem ersten und dem zweiten Endbereich des letzteren ausgelegt ist; und worin die Verbindung dargestellt ist durch das Teil der gebündelten Drähte, die in dem Schlitz aufgenommen sind und das Kugelglied aufgenommen und gelagert ist in der Vertiefung oder Mulde.

15. Elektrodenimplantatanordnung für eine Cochlea nach Anspruch 1, worin sowohl der Elektrodenträger (51) als auch das Positionierglied (52) die Form eines stabilen Stabes aufweisen; das Mittel (54), welches die Schlepp-(51b, 52b) Endbereiche des Elektrodenträgers (51) und des Positioniergliedes (52) miteinander verbindet, enthält eine rohrförmige Halterung (59) mit einem Körper, der ausgestattet ist mit einer internen allgemein axial ausgerichteten Zwischenwand (59c) und definiert auf gegenüberliegenden Seiten der Zwischenwand erste und zweite, im Wesentlichen parallele, Durchgänge (59a, 59b), die sich im Allgemeinen in axialer Richtung durch die rohrförmige Halterung (59) erstrecken, um den Elektrodenträger (51) und in entsprechender Weise das Positionierglied (52) aufzunehmen; das Teil des Körpers (57) der rohrförmigen Halterung (59), welches den ersten Durchgang (59a) bestimmt, ist ständig mit dem Elektrodenträger (51) befestigt für gemeinsame Bewegungen der rohrförmigen Halterung (59) mit dem Elektrodenträger (51); der Teil des Körpers (57) der rohrförmigen Halterung (59), welche den zweiten Durchgang (59b) definiert, ist derart ausgelegt, um eine gleitende Bewegung des Positioniergliedes (52) relativ dazu zu ermöglichen; und die rohrförmige Halterung (59) ist ausgestattet mit Mitteln, die angepasst sind, um nach vollständiger Einführung der Anordnung in die Cochlea aktiviert zu werden, um das Positionierglied (53) in der gebogenen Gestaltung desselben zu immobilisieren gegen rückwärtige Bewegung des Positioniergliedes (52) relativ zu der rohrförmigen Halterung (59) und zur Stabilisierung der Anordnung in der Cochlea.

16. Elektrodenimplantatanordnung für eine Cochlea nach Anspruch 15, worin das Teil des Körpers (57) der rohrförmigen Halterung (59), welches den zweiten Durchgang (59b) definiert, verformbar ist um ein Pfalzen desselben auf das Positionierglied (52) zu ermöglichen und das verformbare Teil des Körpers (57) der rohrförmigen Halterung (59) stellt das aktivierbare Mittel für die Immobilisierung des Positioniergliedes (52) dar.

17. Elektrodenimplantatanordnung für eine Cochlea nach Anspruch 16, worin die rohrförmige Halterung (59) eine Drahtlänge aufweist, die spiralförmig gebogen ist, um sowohl eine äußere Wand des Körpers (57) der rohrförmigen Halterung (59) zu bestimmen als auch die innere Zwischenwand (59c) desselben zwischen den ersten und zweiten Durchgängen.

18. Elektrodenimplantatanordnung für eine Cochlea nach Anspruch 15, worin das Teil des Körpers (57) der rohrförmigen Halterung (59), welches die interne Zwischenwand (59c) desselben definiert, ausgestattet ist mit einer keilförmigen Struktur, die sich von der Zwischenwand in Richtung der Achse des zweiten Durchganges erstreckt, um ineinander zu greifen mit dem Positionierglied (52), wenn Letzteres untergebracht ist in dem zweiten Durchgang, wobei die keilförmige Struktur derart angeordnet ist, um eine Vorwärtsbewegung zu erlauben und eine rückwärtige Bewegung des Positioniergliedes (52) durch den zweiten Durchgang zu blockieren und die keilförmige Struktur stellt das Mittel dar, welches zur Immobilisierung des Positioniergliedes (52) aktivierbar ist.

19. Elektrodenimplantatanordnung für eine Cochlea nach Anspruch 18, worin die keilförmige Struktur ein Sperrglied aufweist, welches von der Zwischenwand hervorspringt und in einem Winkel gegen die Achse des zweiten Durchganges angestellt ist, wobei das Sperrglied einen vorderen Rand aufweist, der nicht auf die Vorwärtsbewegung des Positioniergliedes durch den zweiten Durchgang einwirkt, aber der in der Lage ist, in das Positionierglied zur Blockierung jeglicher rückwärtiger Bewegung des Letzteren einzuschneiden.

20. Elektrodenimplantatanordnung für eine Cochlea nach Anspruch 19, worin die rohrförmige Halterung einen Metallstreifen aufweist, der gewunden ist, um sowohl eine äußere Wand des Körpers der rohrförmigen Halterung als auch die innere Zwischenwand desselben zwischen den ersten und den zweiten Durchgängen zu bestimmen und das Teil des Streifens, welcher die Zwischenwand darstellt, weist eine laterale, winkelig angestellte Ausdehnung auf, die wiederum das Sperrglied darstellt.

21. Elektrodenimplantatanordnung für eine Cochlea nach Anspruch 1, worin der Elektrodenträger (51) die Form eines stabilen Stabes aufweist; das Positionierglied (52) eine elastische, axiale, zusammengepresste Röhre aufweist, wobei deren Länge zwischen deren Führungs- und Schleppendbereichen in ihrem verdichteten Zustand geringfügig größer ist als die Länge der Anordnung der Elektrodenelemente (55) auf dem Elektrodenträger (51), und ein elastisches Spannungsglied erstreckt sich gleitend durch die Röhre und weist erste und zweite Endbereiche auf, die hervorspringen von der Röhre an den Führungs- und Endbereichen in entsprechender Weise; wobei das Mittel zur Verbindung der Führungsendbereiche (51a) des Elektrodenträgers (51) und des Positioniergliedes (52) miteinander eine feste Verbindung zwischen dem ersten Endbereich des Spannungsgliedes und des Führungsendbereiches des Elektrodenträgers (51) enthält; und wobei das Mittel zur Verbindung der Schleppendbereiche des Elektrodenträgers (51) und des Positioniergliedes (52) miteinander eine feste Verbindung zwischen dem Schleppendbereich der Röhre und dem Führungsendbereich des Elektrodenträgers (51) aufweist, und das Mittel zur lösbaren Befestigung des zweiten Endbereiches des Spannungsgliedes mit dem Elektrodenträger (51) außerhalb und etwa rückwärtig zu dem fixierten Schleppendbereich des Röhrchens ist; wobei das Befestigungsmittel, wenn es aktiviert ist mit dem Spannungsmittel, direkt vor der Einführung der Anordnung in die Cochlea, dazu dient, das Positionierungsglied (52) eng angrenzend zu dem Elektrodenträger (51) zu belassen, während es dem Elektrodenträger (51) und auch dem Positionierglied (52) gemeinsam ermöglicht ist, die Krümmung der Cochlea anzunehmen; bei Deaktivierung nach der Einführung der Anordnung in die Cochlea es zum Freigeben des Spannungsgliedes dient, um dem Röhrchen zu erlauben, wenn es versteift durch das Spannungsglied ist, sich auszudehnen und die gebogene Gestaltung anzunehmen und den Elektrodenträger (51) innerhalb der Cochlea so zu verschieben, um eine Positionierung angrenzend zu den Kontaktflächen der Anordnung der Elektrodenelemente zu den Zellen des Spiralganglions zu ermöglichen; und für den Fall einer Reaktivierung dient es dazu mit dem Positionierglied (52) in der gebogenen Gestaltung desselben, das Spannungsglied zu immobilisieren, um das Positionierglied (52) in der gekrümmten Gestaltung für die dadurch erzielte Stabilisierung der Anordnung in der Cochlea beizubehalten.

22. Elektrodenimplantatanordnung für eine Cochlea nach Anspruch 21, worin das Spannungsglied ein Draht aus einem bioverträglichen Metall ist.

23. Elektrodenimplantatanordnung für eine Cochlea nach Anspruch 1, worin sowohl der Elektrodenträger (51) als auch das Positionierglied (52) die Form eines festen Stabes aufweisen, wobei der Elektrodenträger (51) aus einem bioverträglichen Kunststoffmaterial hergestellt ist, welches widerstandsfähig ist gegenüber Aufquellung und Ausdehnung, falls es Wasser ausgesetzt ist, und das Positionierglied (52) aus einem bioverträglichen Kunststoffmaterial hergestellt ist, welches die Eigenschaft hat, aufzuquellen und sich auszudehnen, wenn es dem Wasser ausgesetzt ist; und das Mittel, welches die Führungs- und Schleppendbereiche des Elektrodenträgers (51) in entsprechender Weise verbindet mit den Führungs- und Schleppendbereichen des Positioniermittels (52) weist ein Paar von festen Verbindungen dazwischen auf; wobei während des Einführens der Anordnung in die Cochlea der Elektrodenträger (51) und das Positionierglied (52) dicht beieinander verweilen und gemeinsam die Krümmung der Cochlea annehmen; nach der Einführung, wenn die Anordnung über eine bestimmte Zeitdauer der Wirkung des Wassers im menschlichen Körper ausgesetzt ist, welches flüssig in der Cochlea vorhanden ist, sich das Positionierglied (52) ausdehnt und die gebogene Gestaltung annimmt und verschiebt den Elektrodenträger (51) innerhalb der Cochlea, um die Kontaktflächen der Anordnung der Elektrodenelemente (55) in angrenzende Position zu den Zellen des Spiralganglions zu bringen; und das Positionierglied (52) in der gekrümmten Gestaltung dient zur Immobilisierung der Anordnung zur Stabilisierung derselben in der Cochlea.

24. Elektrodenimplantatanordnung für eine Cochlea nach Anspruch 23, worin die festen Verbindungen zwischen dem Elektrodenträger (51) und dem Positionierglied (52) Formoder Klebeverbindungen sind.

25. Elektrodenimplantatanordnung für eine Cochlea nach Anspruch 1, worin das Mittel (53), welches die Führungsendbereiche des Elektrodenträgers (51) und das Positionierglied (52) miteinander verbindet, ein sperrfreies Kugelgelenk aufweist, beinhaltend ein stabförmiges Teil, welches unterstützt wird von einem, dem Positionierelement (52) und Elektrodenträger (51) und einem korrespondierend gestalteten Muffenteil, unterstützt durch das andere aus Positionierglied (52) und Elektrodenträger (51), wobei das Muffenteil derart aufgebaut ist, um eine gleitende Aufnahme des stabförmigen Teiles zu erzielen, ohne dass die beiden Teile sich gegeneinander sperren.

26. Elektrodenimplantatanordnung für eine Cochlea nach Anspruch 25, worin das Mittel zur Verbindung der Schleppendbereiche des Elektrodenträgers (51) und des Positioniergliedes (52) miteinander ein ringförmiges Glied aus einem bioverträglichen Kunststoffmaterial aufweisen, und einen sich hindurch erstreckenden Durchgang definieren, der derart gestaltet ist, um gemeinsam den Elektrodenträger (51) und das Positionierglied (52) mit einer engen Passung aufzunehmen; und das ringförmige Glied eine Querschnittsausgestaltung aufweist, die eine umlaufende Randoberfläche für den Durchgang bestimmt, welche mittels Reibschluss das Positionierglied (52) zurückhält gegen versehentliche rückwärtige Bewegungen durch das ringförmige Glied und aus der Cochlea heraus, wenn das Positionierglied (52) sich in dem gebogenen Zustand befindet und inder Abwesenheit einer longitudinal und rückwärtig gerichteten Kraft mit ausreichender Größe, die von außen durch den Operateur, auf das Positionierglied (52) aufgebracht wird.

27. Elektrodenimplantatanordnung für eine Cochlea nach Anspruch 26, worin die Querschnittsgestaltung des ringförmigen Gliedes größtenteils D-förmig ist, wobei mit der flachen Wand des D die umlaufende Randoberfläche des Durchganges definiert ist.

28. Elektrodenimplantatanordnung für eine Cochlea nach einem der Ansprüche 25 bis 27, worin das Muffenteil der Verbindung unterstützt wird durch den Elektrodenträger (51) und das Stabteil der Verbindung unterstützt wird durch das Positionierglied (52).

29. Elektrodenimplantatanordnung für eine Cochlea nach Anspruch 28, worin das Muffenteil der Verbindung eine spulenförmige Ausformung des Drahtes aus einem bioverträglichen Metallmaterial aufweist und eingebettet ist in den Führungsendbereich des Elektrodenträgers (51), wobei die spulenförmige Ausfformung des Drahtes eine Achse und einen axialen inneren Hohlraum zur Aufnahme des Stabteiles der Verbindung aufweist und die Achse der spulenförmigen Ausformung des Drahtes schräg angestellt ist innerhalb des Elektrodenträgers (51), nach hinten und aufwärts gerichtet in der Richtung von dem ersten zum zweiten Längsseitenbereich desselben; der Elektrodenträger (51) bereitgestellt ist an dem zweiten Längsseitenbereich desselben mit einer Zugangsöffnung, ausgerichtet und in Verbindung stehend mit dem inneren Hohlraum der spulenförmigen Ausformung des Drahtes; das Stabteil der Verbindung ein stabförmiges Element aus einem bioverträglichen metallischen Material aufweist und nach vorne hervorragt von dem Führungsendbereich des Positioniergliedes (52) und das stabförmige Element schräg angestellt ist relativ zu dem Positionierglied (52), nach vorne und abwärts in der Richtung von dem Positionierglied (52) zu dem Elektrodenträger (51), wenn selbige angrenzend zueinander in der Anordnung positioniert sind; und die Verbindung aufgebaut wird durch das stabförmige Element, welches gleitend in den inneren Hohlraum der spulenförmigen Ausformung des Drahtes durch die Zugangsöffnung eindringt, wobei lediglich eine vorwärtsgerichtete Bewegung des Positioniergliedes (52) eine Schiebekraft verursacht, die durch das stabförmige Element auf die spulenförmige Ausformung des Drahtes wirkt, um eine vorwärtsgerichtete Bewegung des Elektrodenträgers (51) zu verursachen, während eine rückwärtige Bewegung des Positioniergliedes (52) das stabförmige Element zurückzieht von der spulenförmigen Ausformung des Drahtes und den Elektrodenträger (51) stationär belässt.

30. Elektrodenimplantatanordnung für eine Cochlea nach Anspruch 29, worin das Positionierglied (52) einen Führungsdraht (67) aufweist, der darin eingebettet ist und sich über die gesamte Länge des Positioniergliedes (52) erstreckt, wobei der Körper des Führungsdrahtes (67) innerhalb des Positioniergliedes (52) im Wesentlichen im Querschnitt flach dargestellt ist und eine flache Oberfläche desselben liegt dem zweiten Längsseitenbereich des Elektrodenträgers (51) gegenüber, wenn das Positionierglied (52) und der Elektrodenträger (51) in der Anordnung gegenseitig benachbart positioniert sind, wobei der Führungsdraht (67) dabei das Positionierglied (52) damit verstärkt gegen eine Biegung in einer Richtung innerhalb der Ebene des Führungsdrahtes (67), während eine ungehinderte Biegung des Positioniergliedes (52) in einer zu der Ebene des Führungsdrahtes (67) senkrechten Richtung ermöglicht ist, und das stabförmige Element wird durch einen Endbereich des Führungsdrahtes (67) dargestellt, welcher außerhalb des Positioniergliedes (52) positioniert ist.

31. Elektrodenimplantatanordnung für eine Cochlea nach Anspruch 30, worin die Breite des Körpers (57) des Führungsdrahtes (67) ungefähr die Hälfte der Breite des Positioniergliedes (52) in einem seitlichen Bereich des letzteren beträgt der unmittelbar bei dem zweiten Längsseitenbereich des Elektrodenträgers (51) positioniert ist, wenn das Positionierglied (52) und der Elektrodenträger (51) in der Anordnung aneinander angrenzen.

32. Elektrodenimplantatanordnung für eine Cochlea nach Anspruch 30, worin das Teil des Führungsdrahtes (67), der das stabförmige Element darstellt, eine herkömmliche runde Querschnittsausbildung aufweist.

33. Implantatanordnung für eine Cochlea angepasst für die Einführung in die Scala Tympani mit einem Elektrodenträger (51), aufweisend eine Vielzahl von Elektroden (55), und mit Führungs- und Schleppenden (51a, b) worin die Anordnung weiterhin ein Positionierglied (52) aufweist,
**dadurch gekennzeichnet dass**
der Elektrodenträger (51) nicht vorgespannt ist, bevor er in die Scala Tympani eingeführt wird, wobei das Positionierglied (52) angepasst ist, um mit dem Elektrodenträger (51) zumindest während der Einführung an dem Führungsende des Elektrodenträgers (51) zusammengefügt zu werden, um den Elektrodenträger (51) während der Einführung zu führen, und nach der Einführung ist das Positionierglied (52) angepasst, um eine gekrümmte Gestaltung anzunehmen relativ zu den Elektrodenträgern (51), wobei ein mittlerer Bereich des Positioniergliedes (52) zwischen den Führungs- und Schleppenden getrennt ist von dem nächstliegenden mittleren Bereich des Elektrodenträgers (51), um den Elektrodenträger (51) in Richtung auf die radiale innere Wand der Scala Tympani zu drücken, wobei die Anordnung weiterhin Mittel (54) aufweist zur Verbindung des Schleppendes (51b) des Elektrodenträgers (51) mit dem Positionierglied (52), so dass die gebogene Ausgestaltung beibehalten wird.

34. Implantatanordnung für eine Cochlea nach Anspruch 33, worin das Positionierglied (52) anfänglich lediglich an dem Führungsende (52a) mit dem Elektrodenträger (51) befestigt ist, und nach der Einführung die Mittel zur Verbindung des Schleppendes befestigt werden.

35. Implantatanordnung für eine Cochlea nach Anspruch 34, worin das Positionierglied (52) befestigt ist an dem Führungsende (51a) des Elektrodenträgers (51) durch Befestigungsmittel, die sich bei rückwärtiger Bewegung des Positioniermittels ablösen, um zu ermöglichen, dass die Positioniermittel abgelöst werden von dem Elektrodenträger (51), um ein Entfernen des Elektrodenträgers (51) zu erleichtern.

36. Implantatanordnung für eine Cochlea nach Anspruch 35, worin das Befestigungsmittel (53) ein sperrfreies Kugelgelenk ist.

37. Implantatanordnung für eine Cochlea nach Anspruch 33, worin der Elektrodenträger (51) anfänglich an beiden Enden mit dem Positionsglied (52) befestigt ist, wobei sich die Länge des Positioniergliedes (52) im Betrieb vergrößert, um die gebogene Ausgestaltung anzunehmen.

38. Implantatanordnung für eine Cochlea nach Anspruch 37, worin sich die Längenvergrößerung aus dem Aufquellen des Positioniergliedes (52) durch Wasser ergibt.

## Revendications

1. Ensemble formant implant à électrodes cochléaires, auto-positionnant, susceptible d'être implanté dans la rampe tympanique de la cochlée, enroulée en spirale, d'une oreille humaine, afin de stimuler les cellules du ganglion spiral, cet ensemble comprenant:
un support d'électrodes cochléaires (51) en forme de tige, constitué en matière plastique biocompatible et suffisamment souple pour pouvoir s'adapter à la courbure en spirale de la cochlée, ce support d'électrodes (51) possédant des zones terminales avant (51a) et arrière (51b) et sa section transversale allant en rétrécissant de zone terminale arrière (51b) vers la zone terminale avant (51a) ; une série d'électrodes cochléaires (55) supportées par le support d'électrodes (51) en des points respectifs longitudinalement espacés de celui-ci, dans une zone médiane du support d'électrodes (51), entre ses zones terminales avant et arrière, ces électrodes possédant des faces de contact respectives à découvert, sur une première face latérale longitudinale du support d'électrodes (51); et un élément de positionnement en forme de tige (52) ; **caractérisé en ce que** l'élément de positionnement (52) est constitué en matière plastique biocompatible et est suffisamment souple pour pouvoir s'adapter à la courbure en spirale de la cochlée, l'élément de positionnement présentant des zones terminales avant (52a) et arrière (52b) et étant juxtaposé au support d'électrodes (51) et s'étendant le long de celui-ci, sur une deuxième face latérale longitudinale de ce dernier qui est opposée à la première face latérale longitudinale, afin que les zones terminales avant (52a) et arrière (52b) de l'élément de positionnement (52) se trouvent être adjacentes aux zones terminales avant (51a) et arrière (51b), respectivement, du support d'électrodes (51); et des moyens (53, 54) assemblant les zones terminales avant (52a) et arrière (52b) de l'élément de positionnement (52) aux zones terminales avant (51a) et arrière (51b), respectivement, du support d'électrodes (51), afin qu'une zone médiane (52c) de l'élément de positionnement reste séparée de la zone médiane (51c) du support d'électrodes (51) et susceptible d'adopter une configuration arquée par rapport à la zone médiane (51c) du support d'électrodes (51);
après insertion de l'ensemble dans la rampe tympanique au moyen de l'élément de positionnement (52) qui y pénètre et qui suit exactement la courbure de la paroi radialement externe de la cochlée, et le support d'électrodes (51) étant juxtaposé sur l'élément de positionnement (52) et suivant exactement sa courbure, l'élément de positionnement (52), lorsqu'il adopte la configuration arquée à l'encontre de la contrainte de la paroi radialement externe de la cochlée, exerce une force sur le support d'électrodes (51), au niveau des zones d'extrémité avant (51a) et arrière (51b) de celui-ci, pour pousser le support d'électrodes (51) afin que sa première face latérale longitudinale coopère avec la paroi radialement interne de la cochlée, afin de positionner les faces de contact de la série d'électrodes (55) à grande proximité des cellules du ganglion spiral de la cochlée ; et l'assemblage entre la zone terminale arrière du support d'électrodes et la zone terminale arrière (51b) de l'élément de positionnement (52) sert, une fois l'ensemble inséré à l'intérieur de la cochlée et une fois l'élément de positionnement (52) dans sa configuration arquée, à stabiliser l'ensemble dans la cochlée et à le bloquer pour l'empêcher d'effectuer, par inadvertance, un mouvement inverse l'en dégageant.

2. Ensemble formant implant à électrodes cochléaires selon la revendication 1, dans lequel les moyens (54) permettant d'assembler les zones terminales arrière (51b, 52b) du support d'électrodes (51) et l'élément de positionnement (52) les uns aux autres comprennent un raccord possédant un corps (57) présentant des parties périphériques extérieures (57a, 57b) au moins partiellement arquées, des extrémités avant et arrière, et une séparation intérieure (57e) s'étendant d'une extrémité du corps à l'autre afin que le raccord présente à travers lui des premier et deuxième passages (57c, 57d), sensiblement parallèles, afin de recevoir le support d'électrodes (51) et l'élément de positionnement (52), respectivement ; la partie du corps du raccord qui, en liaison avec la séparation (57e), définit le premier ou le deuxième passage (57c, 57d) étant fixée de façon permanente sur, respectivement, le support d'électrodes (51) ou l'élément de positionnement (52) qui s'étend dans le passage ; la partie du corps (57) du raccord qui, en liaison avec la séparation (57e), définit l'autre passage, choisi parmi le premier ou le deuxième, étant dimensionnée pour permettre un mouvement coulissant relatif entre le raccord et l'autre élément, choisi parmi le support d'électrodes (51) et l'élément de positionnement (52), qui s'étend dans l'autre passage ; et la partie du corps (57) du raccord dans laquelle se trouve l'autre passage étant pourvue de moyens permettant à l'autre élément, choisi parmi le support d'électrodes (51) et l'élément de positionnement (52), d'être bloqué pour empêcher son déplacement à travers l'autre passage.

3. Ensemble formant implant à électrodes cochléaires selon la revendication 2, dans lequel le deuxième passage (57d) est dimensionné pour permettre un mouvement coulissant relatif entre le raccord et l'élément de positionnement (52), et dans lequel la partie du corps (57) du raccord dans laquelle se trouve le deuxième passage (57d) est pourvue de moyens permettant de bloquer l'élément de positionnement (52) pour empêcher son déplacement à travers le deuxième passage (57b).

4. Ensemble formant implant à électrodes cochléaires selon la revendication 2 ou 3, dans lequel la partie du corps (57) du raccord dans laquelle se trouve le premier passage (57c) est fixée de façon permanente sur le support d'électrodes (51).

5. Ensemble formant implant à électrodes cochléaires selon la revendication 2 ou 3, dans lequel la partie du corps (57) du raccord dans laquelle se trouve le deuxième passage (57d) est pourvue de moyens du type clavette permettant un mouvement vers l'avant mais empêchant un mouvement vers l'arrière de l'élément de positionnement (52) dans le deuxième passage (57d).

6. Ensemble formant implant à électrodes cochléaires selon la revendication 5, dans lequel les moyens du type clavette (57f) comprennent un élément du type cliquet (57f) supporté par et s'étendant vers l'avant à partir de la partie du corps (57) du raccord dans laquelle se trouve le deuxième passage (57d), cet élément du type cliquet (57f) étant incliné en direction de l'axe du deuxième passage (57d), et présentant un bord avant (57h) qui coopère de façon coulissante avec une première surface de l'élément de positionnement (52) lorsque ce dernier se déplace vers l'avant dans le deuxième passage, le bord avant de l'élément du type cliquet (57f) étant suffisamment pointu pour mordre dans la première surface de l'élément de positionnement (52), bloquer ainsi l'élément de positionnement (52) en l'empêchant de se déplacer vers l'arrière dans le deuxième passage (57d), sensiblement immédiatement après le début de ce mouvement vers l'arrière.

7. Ensemble formant implant à électrodes cochléaires selon la revendication 6, dans lequel l'élément du type cliquet (57f) est supporté par et s'étend vers l'avant, à partir de la séparation (57e), en étant incliné en direction de l'axe du deuxième passage (57d).

8. Ensemble formant implant à électrodes cochléaires selon la revendication 7, dans lequel l'élément du type cliquet (57f) est supporté par la séparation (57e) de façon adjacente à l'extrémité de la séparation qui se trouve le plus en arrière, et s'étend vers l'avant, à partir de celle-ci, à l'intérieur des limites du deuxième passage (57d).

9. Ensemble formant implant à électrodes cochléaires selon la revendication 8, dans lequel l'élément du type cliquet (57f) est soudé sur la séparation (57e).

10. Ensemble formant implant à électrodes cochléaires selon la revendication 8, dans lequel l'élément du type cliquet (57f) est une prolongation de la séparation (57e), ne formant qu'une seule pièce avec elle, au niveau de l'extrémité la plus en arrière de la séparation, et est pliée, à partir de celle-ci, dans le deuxième passage (57d).

11. Ensemble formant implant à électrodes cochléaires selon la revendication 8, dans lequel la partie du corps (57) du raccord dans laquelle se trouve le deuxième passage (57d) présente un bord avant (57i) qui est espacé du bord avant (57h) de l'élément du type cliquet (57e) et coopère de façon coulissante avec une deuxième surface de l'élément de positionnement (52), et qui est suffisamment pointu pour mordre dans la deuxième surface de l'élément de positionnement (52), aidant ainsi à bloquer l'élément de positionnement (52) en l'empêchant de se déplacer vers l'arrière dans le deuxième passage (57d), sensiblement immédiatement après le début de ce mouvement vers l'arrière.

12. Ensemble formant implant à électrodes cochléaires selon la revendication 1, dans lequel le support d'électrodes (51) et l'élément de positionnement (52) présentent chacun la forme d'une tige pleine ; et les moyens (53) d'assemblage des zones terminales avant du support d'électrodes (51) et de l'élément de positionnement (52) les unes aux autres comprennent un joint à rotule (53) comprenant une rotule (53a) et une cavité articulaire (53c) de forme correspondante prévues, chacune, sur le support d'électrodes (51) ou l'élément de positionnement (52).

13. Ensemble formant implant à électrodes cochléaires selon la revendication 12, dans lequel la rotule (53a) du joint (53) est située sur le support d'électrodes (51), et la cavité articulaire (53c) du joint (53) est située sur l'élément de positionnement (52).

14. Ensemble formant implant à électrodes cochléaires selon la revendication 13, dans lequel la rotule (53a) du joint (53) comprend une pluralité de fils métalliques en faisceau, constitués dans un matériau métallique biocompatible, qui sont noyés dans le support d'électrodes (51), dans une première zone terminale de celui-ci, et qui dépassent globalement longitudinalement de la zone terminale avant (51a) du support d'électrodes (51), et les fils métalliques en faisceau, à l'extérieur du support d'électrodes (51), se terminent dans une deuxième zone terminale et supportent une rotule ; la cavité articulaire du joint comprend une fente axiale s'étendant vers l'arrière, ménagée dans la zone terminale avant (52b) de l'élément de positionnement (52), dans un plan axial commun à l'élément de positionnement (52) et au support d'électrodes (51), et un évidement ou une dépression de surface, destiné(e) à recevoir la rotule, prévu(e) sur l'élément de positionnement (52), sur une zone latérale de ce dernier qui est orientée à l'opposé du support d'électrodes (51) et en un point où il/elle enjambe la fente, les dimensions de cette fente étant calculées pour loger la partie des fils métalliques en faisceau située entre les première et deuxième zones terminales de ceux-ci ; et le joint est constitué par le fait que la partie des fils métalliques en faisceau est reçue dans la fente et que la rotule est reçue et logée dans l'évidement ou la dépression.

15. Ensemble formant implant à électrodes cochléaires selon la revendication 1, dans lequel le support d'électrodes (51) et l'élément de positionnement (52) présentent, chacun, la forme d'une tige pleine ; les moyens (54) assemblant les unes aux autres les zones terminales arrière (51b, 52b) du support d'électrodes (51) et de l'élément de positionnement (52) comprennent un raccord tubulaire (59) possédant un corps présentant une séparation intérieure globalement axiale (59c) et définissant, sur les faces opposées de la séparation, des premier et deuxième passages (59a, 59b) sensiblement parallèles, s'étendant à travers le raccord tubulaire (59) et globalement dans le sens axial de celui-ci, afin de recevoir le support d'électrodes (51) et l'élément de positionnement (52), respectivement ; la partie du corps (57) du raccord tubulaire (59) qui définit le premier passage (59a) est fixée de façon permanente au support d'électrodes (51) pour que le raccord tubulaire (59) accompagne le mouvement du support d'électrodes (51) ; les dimensions de la partie du corps (57) du raccord tubulaire (59) qui définit le deuxième passage (59b) sont calculées pour permettre un mouvement coulissant de l'élément de positionnement (52) par rapport à celui-ci ; et le raccord tubulaire (59) est équipé de moyens susceptibles d'être activés, une fois l'ensemble inséré dans la cochlée, afin d'immobiliser l'élément de positionnement (52) dans sa configuration arquée, afin d'empêcher un déplacement inverse de l'élément de positionnement (52) par rapport au raccord tubulaire (59), et stabilisant ainsi l'ensemble dans la cochlée.

16. Ensemble formant implant à électrodes cochléaires selon la revendication 15, dans lequel la partie du corps (57) du raccord tubulaire (59) qui définit le deuxième passage (59b) est déformable pour permettre un sertissage de celle-ci sur l'élément de positionnement (52), et la partie déformable du corps (57) du raccord tubulaire (59) constitue les moyens qui sont susceptibles d'être activés pour immobiliser l'élément de positionnement (52).

17. Ensemble formant implant à électrodes cochléaires selon la revendication 16, dans lequel le raccord tubulaire (59) comprend une longueur de fil métallique enroulé en spirale pour définir une paroi externe du corps (57) du raccord tubulaire (59) et la séparation interne (59c) de celui-ci, située entre les premier et deuxième passages.

18. Ensemble formant implant à électrodes cochléaires selon la revendication 15, dans lequel la partie du corps (57) du raccord tubulaire (59) qui définit la séparation interne (59c) de celui-ci est pourvue d'un élément du type clavette s'étendant à partir de la séparation, en direction de l'axe du deuxième passage, afin de coopérer avec l'élément de positionnement (52) lorsque ce dernier est logé dans le deuxième passage, cet élément du type clavette étant conçu pour permettre un mouvement vers l'avant et pour bloquer un mouvement vers l'arrière de l'élément de positionnement (52) dans le deuxième passage, cet élément du type clavette constituant les moyens susceptibles d'être activés pour immobiliser l'élément de positionnement (52).

19. Ensemble formant implant à électrodes cochléaires selon la revendication 18, dans lequel l'élément du type clavette comprend un élément du type cliquet qui dépasse vers l'avant à partir de la séparation et qui est incliné, en formant un angle, en direction de l'axe du deuxième passage, l'élément du type cliquet présentant un bord avant qui ne gêne pas le mouvement vers l'avant de l'élément de positionnement à travers le deuxième passage, mais qui est susceptible de mordre dans l'élément de positionnement pour empêcher tout déplacement vers l'arrière de celui-ci.

20. Ensemble formant implant à électrodes cochléaires selon la revendication 19, dans lequel le raccord tubulaire comprend une bande de métal enroulé pour définir à la fois une paroi externe du corps du raccord tubulaire et la séparation interne de celui-ci, entre le premier et le deuxième passage, et la partie de cette bande qui constitue la séparation possède un prolongement latéral incliné qui constitue l'élément du type cliquet.

21. Ensemble formant implant à électrodes cochléaires selon la revendication 1, dans lequel le support d'électrodes (51) présente la forme d'une tige pleine ; l'élément de positionnement (52) comprend un tube élastique axialement comprimé, dont la longueur, entre ses zones terminales avant et arrière, à l'état comprimé, est quelque peu supérieure à la longueur de la série d'électrodes (55) présentes sur le support d'électrodes (51), et un élément de tension flexible s'étendant avec possibilité de coulissement à travers le tube et possédant des première et deuxième zones terminales dépassant du tube, au niveau des zones terminales avant et arrière, respectivement, du tube ; les moyens permettant d'assembler les zones terminales avant (51a) du support d'électrodes (51) et l'élément de positionnement (52) comprennent une liaison fixe entre la première zone terminale de l'élement de tension et la zone terminale avant du support d'électrodes (51); et les moyens d'assemblage des zones terminales arrière du support d'électrodes (51) et de l'élément de positionnement (52) comprennent une liaison fixe entre la zone terminale arrière du tube et la zone terminale arrière du support d'électrodes (51), et des moyens permettant de fixer de façon détachable la deuxième zone terminale de l'élément de tension au support d'électrodes (51) à l'extérieur de la zone terminale arrière fixe du tube, et légèrement vers l'arrière de celle-ci ; ces moyens de fixation, lorsqu'ils sont activés, l'élément de tension étant droit, avant l'insertion de l'ensemble dans la cochlée, servant à maintenir l'élément de positionnement (52) juxtaposé de façon ajustée sur le support d'électrodes (51) tout en permettant au support d'électrodes (51) et à l'élément de positionnement de suivre tous deux la courbure de la cochlée ; lorsqu'ils sont désactivés après l'insertion de l'ensemble dans la cochlée, les moyens de fixation servent à dégager l'élément de tension afin de permettre au tube, tout en étant rendu rigide par l'élément de tension, de s'élargir et d'adopter la configuration arquée, et de déplacer le support d'électrodes (51), à l'intérieur de la cochlée, afin de juxtaposer les faces de contact de la série d'électrodes et les cellules du ganglion spiral ; et, lorsqu'ils sont réactivés, l'élément de positionnement (52) se trouvant dans sa configuration arquée, les moyens de fixation servent à immobiliser l'élément de tension afin de maintenir l'élément de positionnement (52) dans la configuration arquée, afin de stabiliser l'ensemble dans la cochlée.

22. Ensemble formant implant à électrodes cochléaires selon la revendication 21, dans lequel l'élément de tension est un fil métallique en métal biocompatible.

23. Ensemble formant implant à électrodes cochléaires selon la revendication 1, dans lequel le support d'électrodes (51) et l'élément de positionnement (52) présentent chacun la forme d'une tige pleine, le support d'électrodes (51) étant constitué dans une matière plastique biocompatible qui résiste au gonflement ou à l'expansion lorsqu'elle est exposée à l'eau, et l'élément de positionnement (52) étant constitué dans une matière plastique biocompatible qui possède la propriété de gonfler ou de se dilater lorsqu'elle est exposée à l'eau ; et les moyens assemblant les zones terminales avant et arrière du support d'électrodes (51) aux zones terminales avant et arrière, respectivement, de l'élément de positionnement (52), comprennent une paire de liaisons fixes situées entre elles ; lors de l'insertion de l'ensemble dans la cochlée, le support d'électrodes (51) et l'élément de positionnement (52) restent juxtaposés de façon ajustée l'un à l'autre et adoptent tous deux la courbure de la cochlée ; après l'insertion, une fois l'ensemble exposé pendant un certain temps à l'action de l'eau présente dans le liquide physiologique qui se trouve dans la cochlée, l'élément de positionnement (52) se dilate et adopte la configuration arquée, et déplace le support d'électrodes (51), à l'intérieur de la cochlée, afin de juxtaposer les faces de contact de la série d'électrodes (55) vers les cellules du ganglion spiral ; et l'élément de positionnement (52), dans la configuration arquée, sert à immobiliser l'ensemble pour stabiliser celui-ci dans la cochlée.

24. Ensemble formant implant à électrodes cochléaires selon la revendication 23, dans lequel les liaisons fixes entre le support d'électrodes (51) et l'élément de positionnement (52) sont des joints moulés ou soudés.

25. Ensemble à électrodes cochléaires selon la revendication 1, dans lequel les moyens (53) permettant d'assembler les zones terminales avant du support d'électrodes (51) et l'élément de positionnement (52) comprennent un accouplement à tige et cavité sans verrouillage, comprenant une partie formant tige montée sur l'élément de positionnement (52) ou le support d'électrodes (51) et une partie formant cavité, de forme correspondante, présente sur l'autre élément, choisi parmi l'élément de positionnement (52) et le support d'électrodes (51), cette partie formant cavité étant conçue pour recevoir, avec possibilité de coulissement, la partie formant tige sans que les deux éléments ne se trouvent verrouillés l'un à l'autre.

26. Ensemble à électrodes cochléaires selon la revendication 25, dans lequel les moyens permettant d'assembler les zones terminales arrière du support d'électrodes (51) et de l'élément de positionnement (52) comprennent un élément en forme d'anneau, constitué en matériau biocompatible et définissant un passage s'étendant à travers lui, dont les dimensions sont conçues pour recevoir, ensemble, de façon serrée, le support d'électrodes (51) et l'élément de positionnement (52) ; et dans lequel l'élément en forme d'anneau présente une section transversale configurée pour définir une surface de délimitation circonférentielle pour le passage qui, lorsque l'élément de positionnement (52) se trouve à l'état arqué, et en l'absence d'une force dirigée longitudinalement et vers l'arrière, d'une importance adaptée, appliquée de l'extérieur par le chirurgien sur l'élément de positionnement (52), empêche, par friction, l'élément de positionnement (52) de se déplacer par inadvertance vers l'arrière, à travers l'élément en forme d'anneau, et de se dégager de la cochlée.

27. Ensemble formant implant à électrodes cochléaires selon la revendication 26, dans lequel la configuration de la section transversale de l'élément en forme d'anneau est une forme globalement de D, la paroi plate du D définissant la surface de délimitation circonférentielle du passage.

28. Ensemble formant implant à électrodes cochléaires selon l'une quelconque des revendications 25 à 27, dans lequel la partie formant cavité du joint est présente sur le support d'électrodes (51) et la partie formant tige du joint est montée sur l'élément de positionnement (52).

29. Ensemble formant implant à électrodes cochléaires selon la revendication 28, dans lequel la partie formant cavité du joint comprend une boucle en fil constituée en matériau métallique biocompatible et noyée dans la zone terminale avant du support d'électrodes (51), cette boucle en fil possédant un axe et un intérieur axialement creux pour recevoir la partie formant tige du joint, et l'axe de la boucle en fil est incliné de façon oblique à l'intérieur du support d'électrodes (51), vers l'arrière et vers le haut, de la première zone latérale longitudinale de celui-ci vers la deuxième ; le support d'électrodes (51) est pourvu, sur sa deuxième zone latérale longitudinale, d'une ouverture d'accès alignée avec l'intérieur creux de la boucle en fil, et communiquant avec lui ; la partie formant tige du joint comprend un élément en forme de tige, constitué en matériau métallique biocompatible et dépassant vers l'avant à partir de la zone terminale avant de l'élément de positionnement (52), et l'élément en forme de tige est incliné de façon oblique par rapport à l'élément de positionnement (52), vers l'avant et vers le bas, dans la direction de l'élément de positionnement (52), en direction du support d'électrodes (51) lorsque ceux-ci sont placés de façon adjacente l'un par rapport à l'autre dans l'ensemble ; et le joint est constitué par le fait que l'élément en forme de tige pénètre en coulissant dans l'intérieur creux de la boucle en fil, par l'ouverture d'accès, seul un déplacement vers l'avant de l'élément de positionnement (52) ayant pour conséquence qu'une force de poussée est exercée par l'élément en forme de tige sur la boucle en fil afin d'entraîner un déplacement vers l'avant du support d'électrodes (51), tandis qu'un déplacement vers l'arrière de l'élément de positionnement (52) entraîne le retrait de l'élément en forme de tige de la boucle en fil et laisse le support d'électrodes (51) stationnaire.

30. Ensemble formant implant à électrodes cochléaires selon la revendication 29, dans lequel l'élément de positionnement (52) possède un fil métallique de guidage (67) noyé à l'intérieur de lui et s'étendant sur toute la longueur de l'élément de positionnement (52), le corps de ce fil métallique de guidage (67), à l'intérieur de l'élément de positionnement (52), présentant une section transversale sensiblement plate et une face plate qui est orientée vers la deuxième zone latérale longitudinale du support d'électrodes (51) lorsque l'élément de positionnement (52) et le support d'électrodes (51) sont logés de façon adjacente dans l'ensemble, le fil métallique de guidage (67) rigidifiant ainsi l'élément de positionnement (52) et l'empêchant de fléchir dans le plan de ce fil métallique de guidage (67), tout en permettant un fléchissement libre de l'élément de positionnement (52) dans une direction perpendiculaire au plan du fil métallique de guidage (67), et l'élément en forme de tige étant constitué par une zone terminale du fil métallique de guidage (67) située à l'extérieur de l'élément de positionnement (52).

31. Ensemble formant implant à électrodes cochléaires selon la revendication 30, dans lequel la largeur du corps (57) du fil métallique de guidage (67) est d'approximativement la moitié de la largeur de l'élément de positionnement (52) dans une zone latérale de ce dernier située à proximité de la deuxième zone latérale longitudinale du support d'électrodes (51) lorsque l'élément de positionnement (52) et le support d'électrodes (51) sont adjacents l'un à l'autre dans cet ensemble.

32. Ensemble formant implant à électrodes cochléaires selon la revendication 30, la partie du fil métallique de guidage (67) qui constitue l'élément en forme de tige est de section transversale conventionnellement ronde.

33. Implant cochléaire susceptible d'être inséré dans la rampe tympanique, comprenant un support d'électrodes (51) comprenant une pluralité d'électrodes (55), et présentant des extrémités avant et arrière (51 a, b), dans lequel l'implant comprend, en outre, un élément de positionnement (52), **caractérisé en ce que** le support d'électrodes (51) n'est pas précontraint avant d'être inséré dans la rampe tympanique, l'élément de positionnement (52) étant susceptible d'être assemblé au support d'électrodes (51), au moins lors de l'insertion au niveau de l'extrémité avant du support d'électrodes (51), afin de guider le support d'électrodes (51) lors de l'insertion et, après l'insertion, l'élément de positionnement (52) étant susceptible d'adopter une configuration arquée par rapport au support d'électrodes (51), une zone médiane de l'élément de positionnement, située entre les extrémités avant et arrière, étant séparée de la zone médiane juxtaposée du support d'électrodes (51), afin de pousser le support d'électrodes (51) en direction de la paroi radialement intérieure de la rampe tympanique, l'implant comprenant, en outre, des moyens (54) pour assembler l'extrémité arrière (51b) du support d'électrodes (51) avec l'élément de positionnement (52) afin de maintenir la configuration arquée.

34. Implant cochléaire selon la revendication 33, dans lequel l'élément de positionnement (52) n'est initialement fixé au support d'électrodes (51) que par l'extrémité avant (52a) et, après insertion, les moyens d'assemblage avec l'extrémité arrière sont fixés.

35. Implant cochléaire selon la revendication 34, dans lequel l'élément de positionnement (52) est fixé à l'extrémité avant (51a) du support d'électrodes (51) par des moyens de fixation qui se détachent lors d'un mouvement inverse des moyens de positionnement, afin de permettre aux moyens de positionnement de se détacher du support d'électrodes (51) pour faciliter le retrait du support d'électrodes (51).

36. Implant cochléaire selon la revendication 35, dans lequel les moyens de fixation (53) sont un accouplement à tige et cavité sans verrouillage.

37. Implant cochléaire selon la revendication 33, dans lequel le support d'électrodes (51) est initialement fixé par ses deux extrémités à l'élément de positionnement (52), la longueur de l'élément de positionnement (52) augmentant, en fonctionnement, pour se placer dans une configuration arquée.

38. Implant cochléaire selon la revendication 37, dans lequel l'allongement est dû au gonflement de l'élément de positionnement (52) lorsqu'il est exposé à l'eau.
